# EUROPEAN PATENT SPECIFICATION

(11) **EP 1 660 655 B1**
(45) Date of publication and mention of the grant of the patent: **02.11.2016**
(21) Application number: 04756045.3
(22) Date of filing: 25.06.2004
(51) Int. Cl.: C12N 15/10

(54) **LOOK-THROUGH MUTAGENESIS**
LOOK-THROUGH-MUTAGENESE
MUTAGENESE DE TYPE LOOK-THROUGH

(30) Priority: 27.06.2003 US 483282 P
(43) Date of publication of application: 31.05.2006
(73) Proprietor: Bioren, LLC, New York, NY 10017-5755 (US)
(72) Inventor: CREA, Roberto, San Mateo, CA 94402 (US)
(74) Representative: Pfizer
(86) International application number: PCT/US2004/020306
(87) International publication number: WO 2005/003345

(56) References cited:
- WO-A-01/44463
- WO-A1-95/23813
- HANES J & PLÜCKTHUN A: "In vitro selection and evolution of functional proteins by using ribosome display" PROCEEDINGS OF THE NATIONAL ACADEMY OF SCIENCES OF USA, NATIONAL ACADEMY OF SCIENCE. WASHINGTON, US, no. 94, 13 May 1997 (1997-05-13), pages 4937-4942, XP002077149 ISSN: 0027-8424

## Description

### Related Applications and Information

This application claims priority to U.S. Provisional Application No. 60/483282, filed on June 27, 2003.

### Background of the Invention

Mutagenesis is a powerful tool in the study of protein structure and function. Mutations can be made in the nucleotide sequence of a cloned gene encoding a protein of interest and the modified gene can be expressed to produce mutants of the protein. By comparing the properties of a wild-type protein and the mutants generated, it is often possible to identify individual amino acids or domains of amino acids that are essential for the structural integrity and/or biochemical function of the protein, such as its binding and/or catalytic activity. The number of mutants that can be generated from a single protein, however, renders it difficult to select mutants that will be informative or have a desired property, even if the selected mutants that encompass the mutations are solely in putatively important regions of a protein (e.g., regions that make up an active site of a protein). For example, the substitution, deletion, or insertion of a particular amino acid may have a local or global effect on the protein.

Previous methods for mutagenizing polypeptides have been either too restrictive, too inclusive, or limited to knocking out protein function rather than to gaining or improving function. For example, a highly restrictive approach is selective or site- directed mutagenesis which is used to identify the presence of a particular functional site or understand the consequences of making a very specified alteration within the functional site. A common application of site directed mutagenesis is in the study of phosphoproteins where an amino acid residue, that would ordinarily be phosphorylated and allow the polypeptide to carry out its function, is altered to confirm the link between phosphorylation and functional activity. This approach is very specific for the polypeptide and residue being studied. Conversely, a highly inclusive approach is saturation or random mutagenesis that is designed to produce a large number of mutations encompassing all possible alterations within a defined region of a gene or protein. This is based on the principle that, by generating essentially all possible variants of a relevant protein domain, the proper arrangement of amino acids is likely to be produced as one of the randomly generated mutants. However, in practice, the vast number of random combinations of mutations generated can prevent the capacity to meaningfully select a desired candidate because of the presence of the so-called "noise" of so many undesired candidates.

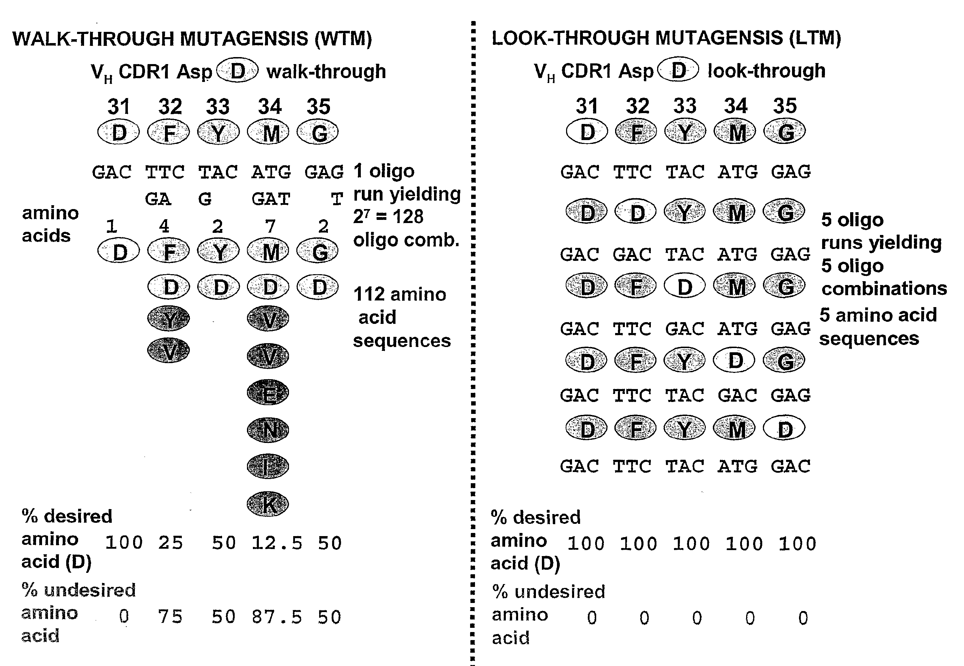

Another approach, referred to as "Walk Through" mutagenesis (see, e.g., U.S. Patent Nos: 5,830,650; 5,798,208) has been used to mutagenize a defined region of a polypeptide by synthesizing a mixture of degenerate oligonucleotides that, statistically, contain a desired set of mutations. However, because degenerate polynucleotide synthesis is employed, Walk-Through mutagenesis yields a number of undesired alterations in addition to the desired set of mutations. For example, to sequentially introduce a mutation across a defined region of only five amino acid positions, a set of over 100 polynucleotide must be made (and screened) (see, e.g., Fig. 6). Accordingly, to make and screen, for example, two or three regions becomes increasingly complex, i.e., requiring the making and screening of 200 to over 300 polynucleotides, respectively, for the presence of only 10 to 15 mutations.

In yet another approach which has been used to mutagenize proteins is alanine scanning mutagenesis, where an alanine residue is "scanned" through a portion of a protein to identify positions where the protein's function is interrupted. However, this approach only looks at loss of protein function by way of substituting a neutral alanine residue at a given position, rather than gain or improvement of function. Thus, it is not a useful approach for generating proteins having improved structure and function.

Accordingly, a need remains for a systematic way to mutagenize a protein for new or improved function.

### Summary of the Invention

The present disclosure pertains to a method of mutagenesis for the generation of novel or improved proteins (or polypeptides) and to libraries of polypeptide analogs and specific polypeptides generated by the methods. The polypeptide targeted for mutagenesis can be a natural, synthetic or engineered polypeptide, including fragments, analogs and mutant forms thereof.

The invention also relates to the following aspects as defined in the following numbered paragraph:
1. A yeast display expression polypeptide library comprising polynucleotides encoding anti-botulinum nerve toxin serotype B (BoNT/B) antibody single chain variable fragment (scFv) analogs, wherein each scFv comprises
   (a) a poly-Gly-Ser linker of SEQ ID NO:7,
   (b) a poly-His tag of SEQ ID NO:8,
   (c) a myc tag of SEQ ID NO:9
   (d) a VH of SEQ ID NO:10 and VL of SEQ ID NO:11 wherein the complementarity determining region (CDR) residues are substituted at each amino acid position within the six CDRs, the polynucleotides collectively representing all possible variant combinations according to the following criteria:
      i) each polynucleotide contains at each codon position in each of the CDRs, either a codon required for the wild type amino acid residue of the polypeptide or a codon for the predetermined amino acid residue, and
      ii) each polynucleotide contains no more than one codon for the predetermined amino acid residue in each CDR; wherein
         the predetermined amino acid residue is selected from the group consisting of Alanine, Leucine, Serine, Aspartic acid, Glutamine, Lysine, Histidine, Tyrosine and Proline, and wherein the anti-BoNT/B scFv is expressed as a fusion partner with Aga2 protein.

In one disclosure, the method comprises introducing a predetermined amino acid into essentially every position within a defined region (or several different regions) of the amino acid sequence of a polypeptide. A polypeptide library is generated containing polypeptide analogs which individually have no more than one predetermined amino acid, but which collectively have the predetermined amino acid in every position within the defined region(s). The method can be referred to as "look- through" mutagenesis because, in effect, a single, predetermined amino acid (and only the predetermined amino acid) is substituted position-by-position throughout one or more defined region(s) of a polypeptide. Thus, the disclosure allows one to "look- through" the structural and functional consequences of separately substituting a predetermined amino acid at each amino acid position within a defined region of the polypeptide, thereby segregating a specific protein chemistry to the defined region without any interference or "noise" from the generation of unwanted polypeptide analogs (i.e., analogs containing amino acid substitutions other than those that follow the "look-through" scheme) (see, for example, Fig. 6).

Accordingly, the present disclosure allows for highly efficient and accurate systematic evaluation of the role of a specific amino acid change in one or more defined regions of a polypeptide. This becomes particularly important when evaluating (by mutating) two or more defined regions, such that the number of polypeptide analogs required greatly increases and, thus, the presence of undesired analogs also increases. The present disclosure obviates this problem by completely eliminating undesired analogs and, thus, the potential that any changes in protein structure or function observed are the result of anything but substitution of the predetermined amino acid. Thus, the effect of segregating a specific protein chemistry to even multiple regions with a protein can be studied with high accuracy and efficiency. Importantly, this includes studying how mutagenesis can effect the interaction of such regions, thereby improving the overall structure and function of the protein.

In a particular embodiment of the disclosure, the library of polypeptide analogs is generated and screened by first synthesizing individual polynucleotides encoding a defined region or regions of a polypeptide where, collectively, the polynucleotides represent all possible variant polynucleotides according to the look-through criteria described herein. The variant polynucleotides are expressed, for example, using in vitro transcription and translation and/or using a display technology, such as ribosome display, phage display, bacterial display, yeast display, arrayed display or any other suitable display system known in the art.

The expressed polypeptides are then screened and selected using functional assays, such as binding assays or enzymatic/catalytic assays. In one disclosure, the polypeptides are expressed in association with the polynucleotide that encodes the polypeptide, thereby allowing for identification of the polynucleotide sequence that encodes the polypeptide. In yet another disclosure, the polypeptides are directly synthesized using protein chemistry.

Thus, the present disclosure provides a method of mutagenesis that can be used to generate libraries of polypeptide analogs that are of a practical size for screening, in part, because the libraries are devoid of any undesired analog polypeptides or so-called noise. The method can be used to study the role of specific amino acids in polypeptide structure and function and to develop new or improved polypeptides such as antibodies, binding fragments or analogs thereof, single chain antibodies, catalytic antibodies, enzymes, and ligands. In addition, the method can be performed with the benefit of a priori information, e.g., via computer modeling, that can be used to select an initial subset of polypeptide analogs to be produced and studied using "look through" mutagenesis.

Other advantages and aspects of the present invention will be readily apparent from the following description and Examples.

### Brief Description of the Figures

Figure 1 illustrates exemplary defined regions (or D regions) that can be examined using Look-Through mutagenesis (LTM) and functional assays for identifying desired polypeptide analogs from such D regions.
Figure 2 illustrates the use of LTM within three defined regions in an antibody variable region (i.e., CDR1, CDR2, and CDR3 of an antibody heavy chain variable region). The light chain variable region can be similarly explored, either alone or in combination with the heavy chain variable region. For convenience in subsequent screening assays, the heavy chain variable region can be explored in the context of a single chain antibody (sFv) as shown.
Figure 3 illustrates the use of LTM within a defined region (i.e., positions 31-25 of CDR1) of a heavy chain variable region.
Figure 4 illustrates the use of LTM within a defined region (i.e., positions 55-68 of CDR2) of a heavy chain variable region.
Figure 5 illustrates the use of LTM within a defined region (i.e., positions 101- 111 of CDR3) of a heavy chain variable region.
Figure 6 illustrates the advantages of LTM as compared to Walk-Through mutagenesis. LTM of a representative defined region, i.e., CDR1 of an antibody heavy chain variable region, results in the sequential alteration of each amino acid position through the defined region, without introducing any undesired amino acid residues or so- called noise.
Figure 7 illustrates the integration of three defined regions of a protein back into an overall protein context following Look-Though mutagenesis, in particular, the integration of all three CDRs of an antibody heavy chain variable region into a single chain antibody format following Look-Though mutagenesis.
Figure 8 illustrates the use of polymerase chain reaction (PCR) to build defined regions of an antibody heavy and light chain subjected to LTM into a larger gene context.

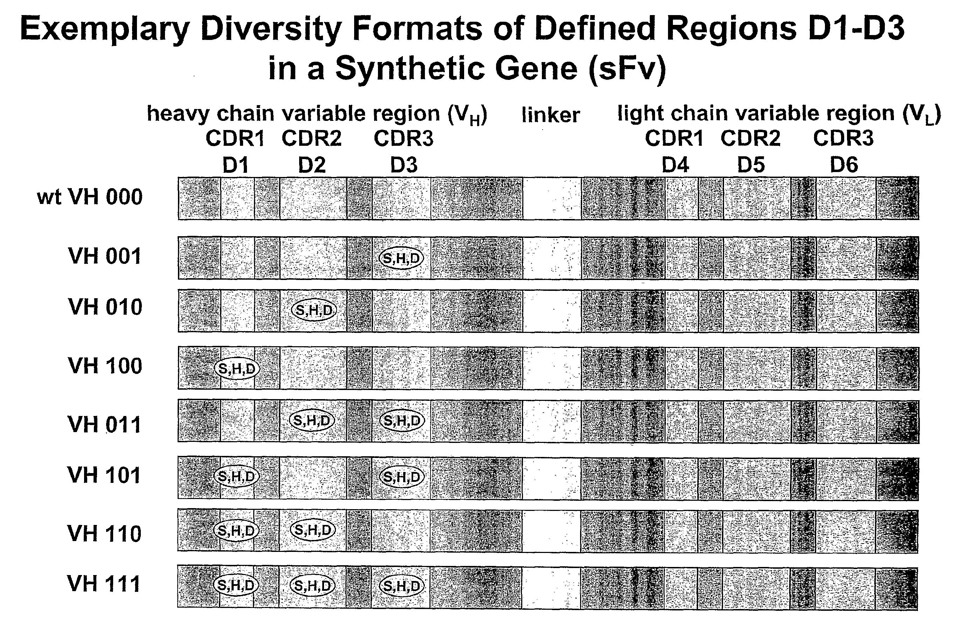

Figure 9 illustrates exemplary diversity formats of each of the CDRs in an antibody variable region for obtaining a catalytic site comprising, e.g., a serine, histidine, and/or aspartic acid and how they can be arrayed.
Figure 10 illustrates the integration of all six CDRs of a binding region of an antibody that have been subjected to LTM and the resultant diversity if one predetermined amino acid residue or twenty different predetermined amino acid residues are used.

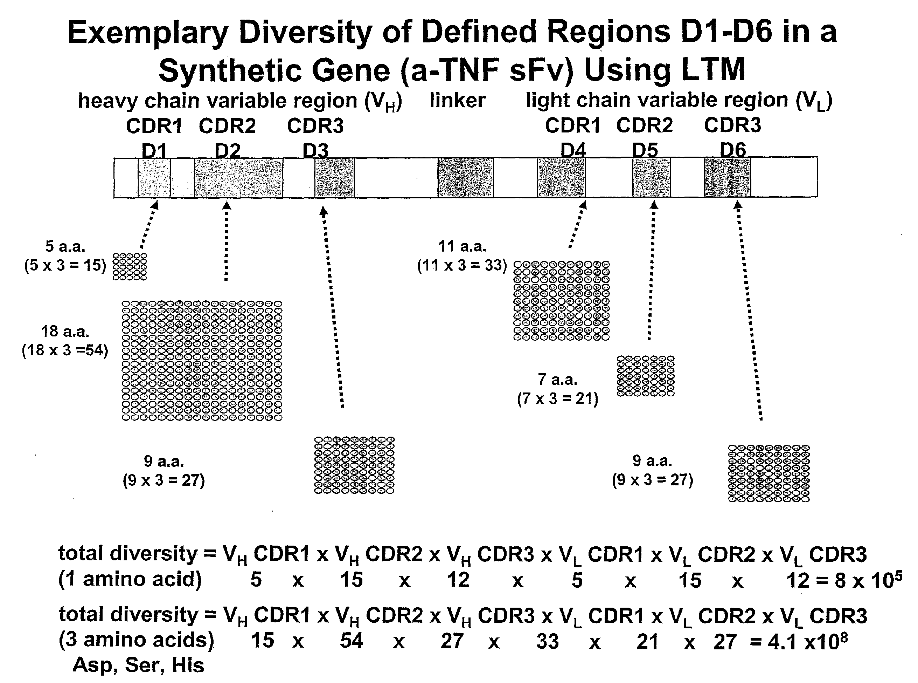

Figure 11 illustrates the integration of all six CDRs of a binding region of an anti-TNF single chain antibody (sFv) that have been subjected to LTM and the resultant diversity if one predetermined amino acid residue or three different predetermined amino acid residues are used.

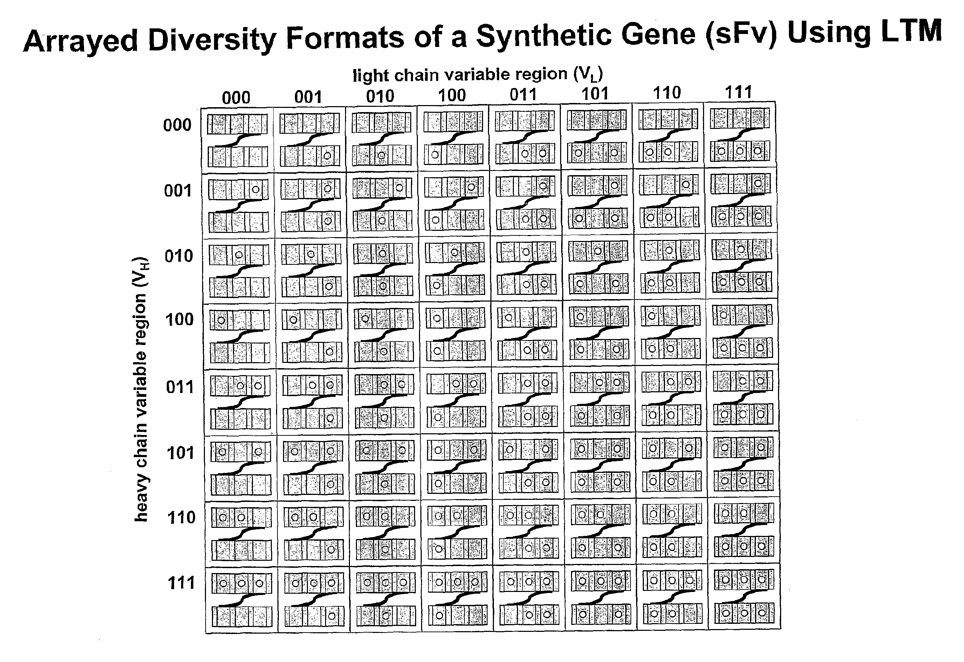

Figure 12 illustrates an arrayed library representing some of the possible polypeptide analogs of the six CDRs of an antibody binding region that can be achieved using LTM.

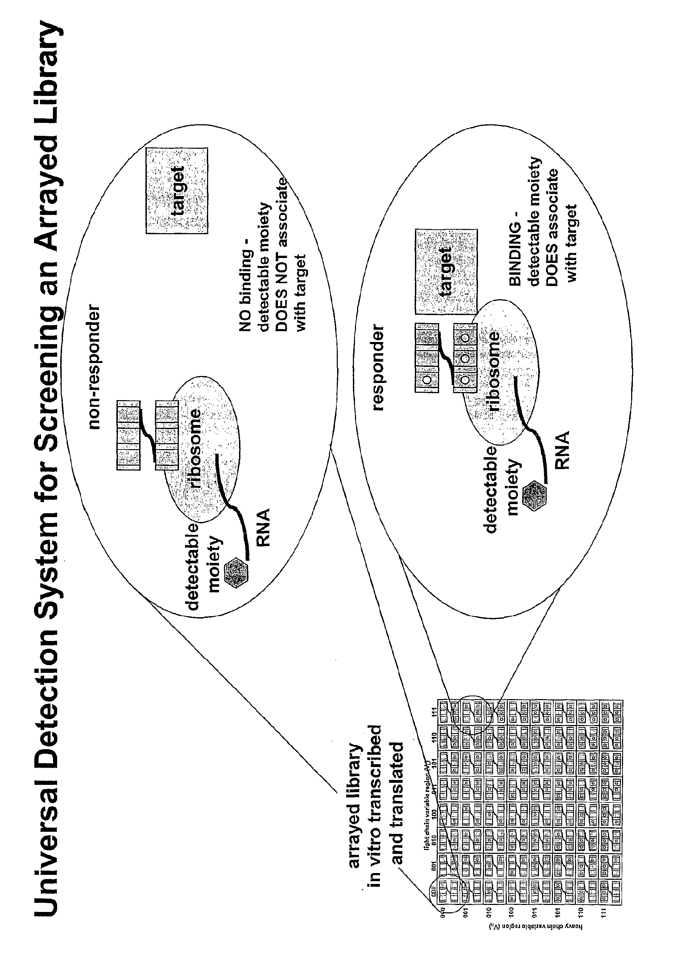

Figure 13 illustrates the screening of an arrayed expression library using cell- free ribosome display.
Figure 14 illustrates the combinatorial chemistry explored when the binding region of an antibody variable region (i.e., all six CDRs) is subjected to LTM.
Figure 15 shows the sequence of the variable region (in single chain format) of several representative anti-TNF binding molecules that can be subjected to LTM.
Figure 16 shows a schematic for carrying out the protease selection assay for screening catalytic candidates of LTM.
Figure 17 shows a schematic detailing the mechanism (and advantages) of the protease selection assay when carried out in bacterial cells.

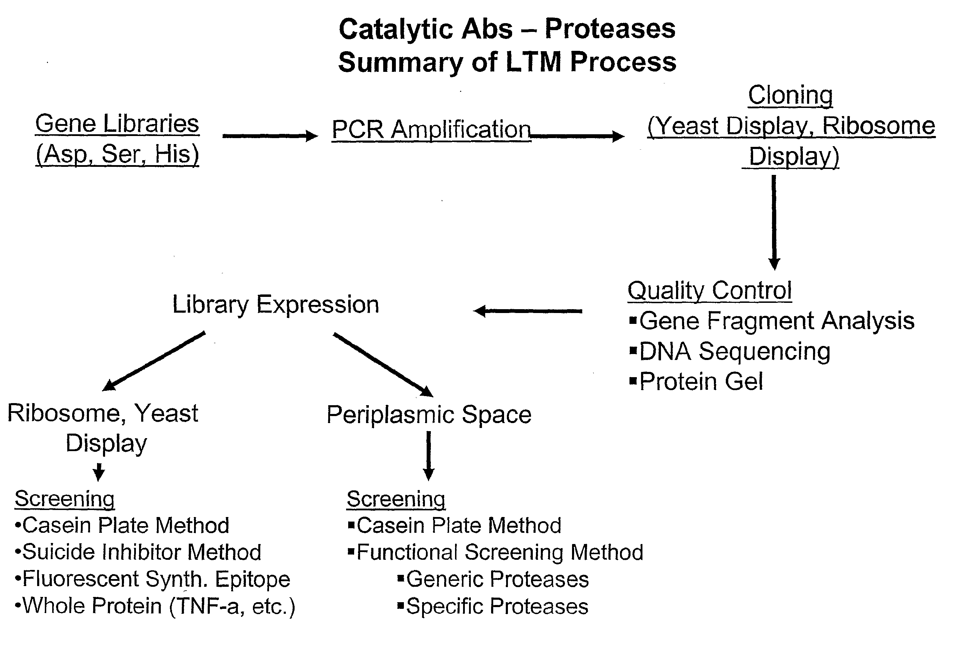

Figure 18 shows a flowchart detailing the mechanics of screening gene libraries for catalytic activity, for example, catalytic antibody activity, using either ribosome or yeast display.
Figure 19 shows a schematic for carrying out LTM where a priori information (e.g., computer modeling information) and empirical information (assay results) can be coordinated for more efficient molecule design and development. This guided approach of LTM is referred to as "guide-through" mutagenesis.
Figure 20 shows a hypothetical V_{H} CDR3 wild-type sequence (uppermost shaded ovals) and the resulting sequences in LTM His substitution (in open ovals) library members. The individual LTM His substitutions are encoded by individual oligonucleotides, e.g., oligonucleotides synthesized in a high-throughput fashion. LTM subset libraries for the other (LTM) amino acid substitutions in this CDR domain are constructed in a similar fashion.

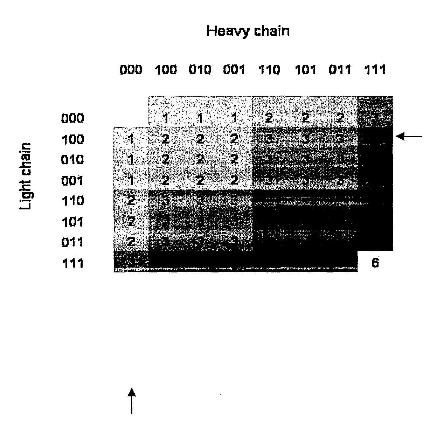

Figure 21 illustrates the generation of scFv libraries. On the top row of the x axis and the far left column of the y axes the three digits represent the 3 CDRs on each of the light and heavy chains. A "0" indicates a wild-type CDR sequence, whereas a "1" indicates an LTM mutated CDR. The number on the grid indicates the complexity of the subset library. For example in the uppermost left corner of the matrix is a "0" where the corresponding x and y axes are "000" and "000" indicating that none of the CDR in either the V_{H} and V_{L} are LTM mutated respectively. In moving one row over from the "0" corner to the neighboring "1" grid position would be designated by x-axis "100" and y-axis "000" indicating that V_{H} CDRI is mutated while all V_{L} CDR remain wild type. Thus, in likewise fashion, a grid numbering of "4" would mean that there are four CDRs simultaneously mutated. Initially the seven V_{H} and seven V chains (indicated by the arrows) are made using SOE-PCR. The V_{H} and V_{L} chains are then amplified and mixed and matched by mega primer to generate all the remaining V_{L}-V_{H} combinations in one step.

### Detailed Description of the Invention

In order to provide a clear understanding of the specification and claims, the following definitions are provided below.

### Definitions

As used herein the term "analog" refers to a variant or mutant polypeptide (or a nucleic acid encoding such a polypeptide) having one or more amino acid substitutions.

The term "binding molecule" refers to any binding molecule, including proteins, polypeptides, peptides, and small molecules, that bind to a substrate or target. In one embodiment of the invention, the binding molecule is an antibody or binding fragment thereof (e.g., a Fab fragment), single domain antibody, single chain antibody (e.g., sFv), or peptide capable of binding a ligand.

The term "defined region" refers to a selected region of a polypeptide. Typically, the defined region includes all or a portion of a functional site, e.g., the binding site of a ligand, the binding site of a binding molecule or receptor, or a catalytic site. The defined region may also include multiple portions of a functional site. For example, the defined region can include all, a portion, or multiple portions of a complementarity determining region (CDR) or a complete heavy and or light chain variable region (NR) of an antibody. Thus, a functional site may include a single or multiple defined regions that contribute to the functional activity of the molecule.

The term "library" refers to two or more molecules mutagenized according to the method of the disclosure. The molecules of the library can be in the form of polynucleotides, polypeptides, polynucleotides and polypeptides, polynucleotides and polypeptides in a cell free extract, or as polynucleotides and/or polypeptides in the context of a phage, prokaryotic cells, or in eukaryotic cells.

The term "mutagenizing" refers to the alteration of an amino acid sequence. This can be achieved by altering or producing a nucleic acid (polynucleotide) capable of encoding the altered amino acid sequence, or by the direct synthesis of an altered polypeptide using protein chemistry.

The term "polynucleotide(s)" refers to nucleic acids such as DNA molecules and RNA molecules and analogs thereof (e.g., DNA or RNA generated using nucleotide analogs or using nucleic acid chemistry). As desired, the polynucleotides may be made synthetically, e.g., using art-recognized nucleic acid chemistry or enzymatically using, e.g., a polymerase. Typical modifications include methylation, biotinylation, and other art-known modifications. In addition, the nucleic acid molecule can be single-stranded or double-stranded and, where desired, linked or associated (e.g., covalently or non- covalently) to a detectable moiety.

The term "variant polynucleotide" refers to a polynucleotide encoding a corresponding polypeptide analog (or portion thereof) of the disclosure. Thus, variant polynucleotides contain one or more codons that have been changed to result in expression of a different amino acid. The term "polypeptide(s)" refers to two or more amino acids joined by a peptide bond, e.g., peptides (e.g., from 2 to -50 amino acid residues), as well as longer peptide sequences e.g., protein sequences which typically comprises amino acid sequences from as few as 50 amino acid residues to more than 1,000 amino acid residues.

The term "pooling" refers to the combining of polynucleotide variants or polypeptide analogs to form libraries representing the Look-Through mutagenesis of an entire polypeptide region. The molecules may be in the form of a polynucleotide and/or polypeptide and may coexist in the form of a sublibrary, as molecules on a solid support, as molecules in solution, and/or as molecules in one or more organisms (e.g., phage, prokaryotic cells, or eukaryotic cells).
The term "predetermined amino acid" refers to an amino acid residue selected for substitution at each position within a defined region of a polypeptide to be mutagenized. This does not include position(s) within the region that already (e.g., naturally) contain the predetermined amino acid and, thus, which need not be substituted with the predetermined amino acid. Accordingly, each polypeptide analog generated in accordance with the present disclosure contains no more than one "predetermined amino acid" residue in a given defined region. However, collectively, the library of polypeptide analogs generated contains the predetermined amino acid at each position within the region being mutagenized. Typically, a predetermined amino acid is selected for a particular size or chemistry usually associated with the side group of the amino acid. Suitable predetermined amino acids include, for example, glycine and alanine (sterically small); serine, threonine, and cysteine (nucleophilic); valine, leucine, isoleucine, methionine, and proline (hydrophobic); phenylalanine, tyrosine, and tryptophan (aromatic); aspartate and glutamate (acidic); asparagine, glutamine, and histidine (amide); and lysine and arginine (basic).

### Detailed Description

The study of proteins has revealed that certain amino acids play a crucial role in their structure and function. For example, it appears that only a discrete number of amino acids participate in the binding of an antibody to an antigen or are involved in the catalytic event of an enzyme.

Though it is clear that certain amino acids are critical to the activity or function of proteins, it is difficult to identify which amino acids are involved, how they are involved, and what substitutions can improve the protein's structure or function. In part, this is due to the complexity of the spatial configuration of amino acid side chains in polypeptides and the interrelationship of different portions of the polypeptide that contribute to form a functional site. For example, the interrelationship between the six CDRs of the variable heavy and light chain regions of an antibody contribute to the antigen or ligand-binding pocket.

Previous mutagenesis methods, such as selective (site-directed) mutagenesis and saturation mutagenesis, are of limited utility for the study of protein structure and function in view of the enormous number of possible variations in complex polypeptides. This is especially true given that desirable combinations are often accompanied by the presence of vast amounts of undesirable combinations or so-called noise.

The method of this disclosure provides a systematic, practical, and highly accurate approach for evaluating the role of particular amino acids and their position, within a defined region of a polypeptide, in the structure or function of the polypeptide and, thus, for producing improved polypeptides.

### 1. Selecting a Defined Region

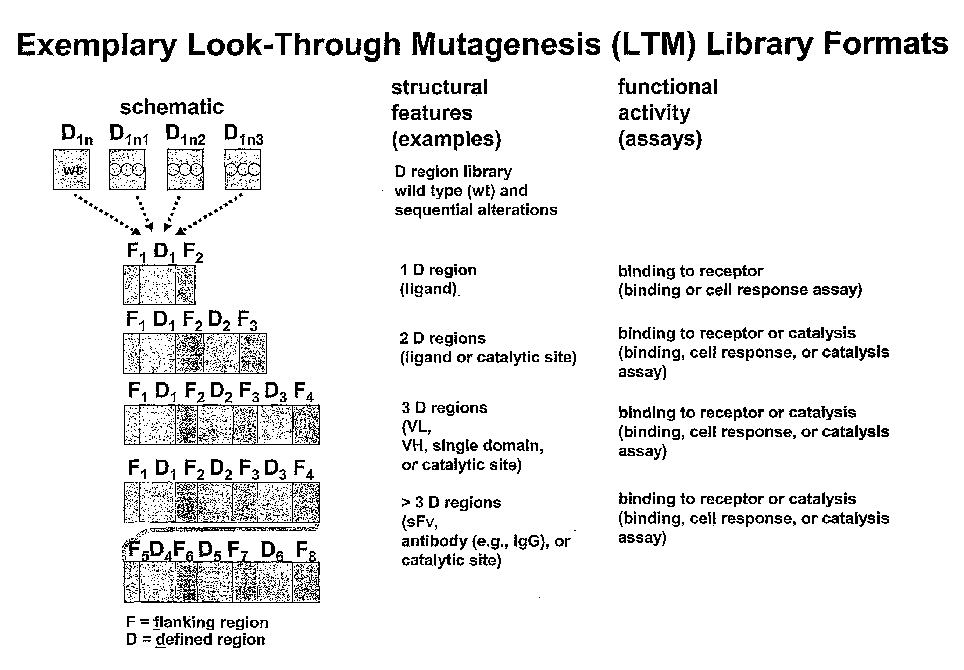

In accordance with the present disclosure, a defined region or regions within a protein are selected for mutagenesis. Typically, the regions are believed to be important to the protein's structure or function (see, e.g., Fig. 1). This can be deduced, for example, from what structural and/or functional aspects are known or can be deduced from comparing the defined region(s) to what is known from the study of other proteins, and may be aided by modeling information. For example, the defined region can be one that has a role in a functional site, e.g., in binding, catalysis, or another function. In one disclosure, the defined region is a hypervariable region or complementarity determining region (CDR) of an antigen binding molecule. In another disclosure, the defined region is a portion of a complementarity determining region (CDR). In other disclosures, two or more defined regions, e.g., CDRs or portions thereof, are selected for mutagenesis.

### 2. Selecting a Predetermined Amino Acid Residue

The amino acid residue chosen for substitution within the defined region(s) is generally selected from those known to be involved in the structure or function of interest. The twenty naturally occurring amino acids differ with respect to their side chain. Each side chain is responsible for chemical properties that make each amino acid unique. For the purpose of altering binding or creating new binding affinities, any of the twenty naturally occurring amino acids generally can be selected. Thus, previous methods of mutagenesis, which created vast numbers of analogs for every substitution, were impractical for evaluating the effect on protein binding of substitution each of the twenty amino acids. In contrast, the methods of the present disclosure creates a practical number of analogs for each amino acid substitution and, thus, allows for the evaluation of a greater variety of protein chemistries within a segregated region or regions of a protein.

In contrast to protein binding, only a subset of amino acid residues typically participate in enzymatic or catalytic events. For example, from the chemical properties of the side chains, only a selected number of natural amino acids preferentially participate in catalytic events. These amino acids belong to the group of polar and neutral amino acids such as Ser, Thr, Asn, Gin, Tyr, and Cys, the group of charged amino acids, Asp and Glu, Lys and Arg, and especially the amino acid His. Other polar and neutral side chains are those of Cys, Ser, Thr, Asn, Gin and Tyr. Gly is also considered to be a borderline member of this group. Ser and Thr play an important role in forming hydrogen bonds. Thr has an additional asymmetry at the beta carbon, therefore only one of the stereoisomers is used. The acid amide Gln and Asn can also for hydrogen bonds, the amido groups functioning as hydrogen donors and the carbonyl groups functioning as acceptors. Gin has one more CH2 group than Asn which renders the polar group more flexible and reduces its interaction with the main chain. Tyr has a very polar hydroxyl group (phenolic OH) that can dissociate at high pH values. Tyr behaves somewhat like a charged side chain; its hydrogen bonds are rather strong.

Neutral polar acids are found at the surface as well as inside protein molecules. As internal residues, they usually form hydrogen bonds with each other or with the polypeptide backbone. Cys can form disulfide bridges.
Histidine (His) has a heterocyclic aromatic side chain with a pK value of 6.0. In the physiological pH range, its imidazole ring can be either uncharged or charged, after taking up a hydrogen ion from the solution. Since these two states are readily available, His is quite suitable for catalyzing chemical reactions. It is found in most of the active centers of enzymes, for example, serine proteases.

Asp and Glu are negatively charged at physiological pH. Because of their short side chain, the carboxyl group of Asp is rather rigid with respect to the main chain. This may be the reason why the carboxyl group in many catalytic sites is provided by Asp and not by Glu. Charged acids are generally found at the surface of a polypeptide.

In addition, Lys and Arg are found at the surface. They have long and flexible side chains presenting multiple rotamers of similar energies, hi several cases, Lys and Arg take part in forming internal salt bridges or they help in catalysis. Because of their exposure at the surface of the polypeptide, Lys is a residue more frequently recognized by enzymes that either modify the side chain or cleave the peptide chain at the carbonyl end of Lys residues.

While the side group chemistry of an amino acid can guide the selection of a predetermined amino acid residue, the lack of a desired side group chemistry can be a criterion for excluding an amino acid residue for use as the predetermined amino acid. For example, sterically small and chemically neutral amino acids, such as alanine, can be excluded from Look-Through mutagenesis for lacking a desired chemistry.

### 3. Synthesizing Polypeptide Analog Libraries

In one embodiment, a library of polypeptide analogs is generated for screening by synthesizing individual oligonucleotides that encode the defined region of the polypeptide and have no more than one codon for the predetermined amino acid. This is accomplished by incorporating, at each codon position within the oligonucleotide either the codon required for synthesis of the wild-type polypeptide or a codon for the predetermined amino acid. This differs from the oligonucleotides produced in saturation mutagenesis, random mutagenesis, or walk-through mutagenesis in that, for each oligonucleotide, only one mutation, as opposed to multiple mutations is made.

The oligonucleotides can be produced individually and then mixed or pooled as desired. When the codon of the wild type sequence and the codon for the predetermined amino acid are the same, no substitution is made.

Accordingly, the number of amino acid positions within the defined region will determine the maximum number of oligonucleotides made. For example, if five codon positions are altered with the predetermined amino acid, then five polynucleotides plus one polynucleotide representing the wild-type amino acid sequence are synthesized. Two or more regions can simultaneously be altered.
The mixture of oligonucleotides for generation of the library can be synthesized readily by known methods for DNA synthesis. The preferred method involves use of solid phase beta-cyanoethyl phosphoramidite chemistry. See U.S. Pat. No. 4,725,677. For convenience, an instrument for automated DNA synthesis can be used containing specified reagent vessels of nucleotides. The polynucleotides may also be synthesized to contain restriction sites or primer hybridization sites to facilitate the introduction or assembly of the polynucleotides representing, e.g., a defined region, into a larger gene context.

The synthesized polynucleotides can be inserted into a larger gene context of the polypeptide being mutagenized by using standard genetic engineering techniques. For example, the polynucleotides can be made to contain flanking recognition sites for restriction enzymes. See Crea, R., U.S. Pat. No. 4,888,286. The recognition sites are designed to correspond to recognition sites that either exist naturally or are introduced in the gene proximate to the DNA encoding the region. After conversion into double stranded form, the polynucleotides are ligated into the gene by standard techniques. By means of an appropriate vector (including, e.g., phage vectors, plasmids) the genes can be introduced into a cell-free extract, phage, prokaryotic cell, or eukaryotic cell suitable for expression of the mutant polypeptides.

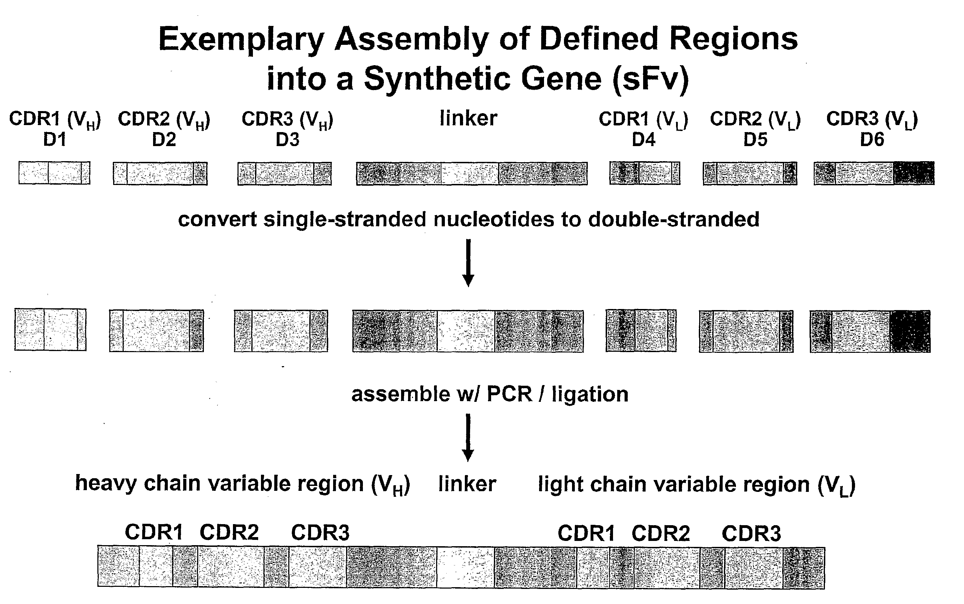

In cases where the amino acid sequence of the polypeptide to be mutagenized is known or where the DNA sequence is known, gene synthesis is a possible approach. For example, partially overlapping polynucleotides, typically about 20-60 nucleotides in length can be designed. The internal polynucleotides are then phosphorylated annealed to their complementary partner to give a double-stranded DNA molecule with single- stranded extensions useful for further annealing. The annealed pairs can then be mixed together and ligated to form a full-length double-stranded molecule (see, e.g., Fig. 8). Convenient restriction sites can be designed near the ends of the synthetic gene for cloning into a suitable vector. The full-length molecules can be cleaved with those restriction enzymes and ligated into a suitable vector. Convenient restriction sites can also be incorporated into the sequence of the synthetic gene to facilitate introduction of mutagenic cassettes.

As an alternative to synthesizing polynucleotides representing the full-length double-stranded gene, polynucleotides which partially overlap at their 3' ends (i.e., with complementary 3' ends) can be assembled into a gapped structure and then filled in with a suitable polymerase to make a full length double-stranded gene. Typically, the overlapping polynucleotides are from 40-90 nucleotides in length. The extended polynucleotides are then ligated. Convenient restriction sites can be introduced at the ends and/or internally for cloning purposes. Following digestion with an appropriate restriction enzyme or enzymes, the gene fragment is ligated into a suitable vector. Alternatively, the gene fragment can be blunt end ligated into an appropriate vector.

In these approaches, if convenient restriction sites are available (naturally or engineered) following gene assembly, the degenerate polynucleotides can be introduced subsequently by cloning the cassette into an appropriate vector. Alternatively, the degenerate polynucleotides can be incorporated at the stage of gene assembly. For example, when both strands of the gene are fully chemically synthesized, overlapping and complementary degenerate polynucleotides can be produced. Complementary pairs will anneal with each other.

When partially overlapping polynucleotides are used in the gene assembly, a set of degenerate nucleotides can also be directly incorporated in place of one of the polynucleotides. The appropriate complementary strand is synthesized during the extension reaction from a partially complementary polynucleotide from the other strand by enzymatic extension with a polymerase. Incorporation of the degenerate polynucleotides at the stage of synthesis also simplifies cloning where more than one domain or defined region of a gene is mutagenized.

In another approach, the gene of interest is present on a single stranded plasmid. For example, the gene can be cloned into a phage vector or a vector with a filamentous phage origin of replication that allows propagation of single-stranded molecules with the use of a helper phage. The single-stranded template can be annealed with a set of degenerate polynucleotides representing the desired mutations and elongated and ligated, thus incorporating each analog strand into a population of molecules that can be introduced into an appropriate host (Sayers, J. R. et al., Nucleic Acids Res. 16: 791-802 (1988)). This approach can circumvent multiple cloning steps where multiple domains are selected for mutagenesis.

Polymerase chain reaction (PCR) methodology can also be used to incorporate polynucleotides into a gene. For example, the polynucleotides themselves can be used as primers for extension. In this approach, polynucleotides encoding the mutagenic cassettes corresponding to the defined region (or portion thereof) are complementary to each other, at least in part, and can be extended to form a large gene cassette using a polymerase, e.g., using PCR amplification.

The size of the library will vary depending upon the length and number of regions and amino acids within a region that are mutagenized. Preferably, the library will be designed to contain less than 10¹⁵, 10¹⁴, 10¹³, 10¹², 10¹¹, 10¹⁰, 10⁹, 10⁸, 10⁷, and more preferably, 10⁶ polypeptide analogs or less.

The description above has centered on the mutagenesis of polypeptides and libraries of polypeptides by altering the polynucleotide that encodes the corresponding polypeptide. It is understood, however, that the scope of the disclosure also encompasses methods of mutagenizing polypeptides by direct synthesis of the desired polypeptide analogs using protein chemistry. In carrying out this approach, the resultant polypeptides still incorporate the features of the disclosure except that the use of a polynucleotide intermediate is eliminated.

For the libraries described above, whether in the form of polynucleotides and/or corresponding polypeptides, it is understood that the libraries may be also attached to a solid support, such as a microchip, and preferably arrayed, using art recognized techniques.

### 4. Expression and Screening Systems

Libraries of polynucleotides generated by any of the above techniques or other suitable techniques can be expressed and screened to identify polypeptide analogs having desired structure and/or activity. Expression of the polypeptide analogs can be carried out using any suitable expression display system known in the art including, but not limited to, cell-free extract display systems (e.g., ribosome display and arrayed (e.g., microarrayed or macroarrayed) display systems), bacterial display systems, phage display systems, prokaryotic cells, and/or eukaryotic cells (e.g., yeast display systems).

In one disclosure, the polynucleotides are engineered to serve as templates that can be expressed in a cell free extract. Vectors and extracts as described, for example in U.S. Patent Nos. 5,324,637; 5,492,817; 5,665,563, can be used and many are commercially available. Ribosome display and other cell-free techniques for linking a polynucleotide (i.e., a genotype) to a polypeptide (i.e., a phenotype) can be used, e.g., Profusion™ (see, e.g., U.S. Patent Nos. 6,348,315; 6,261,804; 6,258,558; and 6,214,553).

Alternatively, the polynucleotides of the disclosure can be expressed in a convenient E. coli expression system, such as that described by Pluckthun and Skerra. (Pluckthun, A. and Skerra, A., Meth. Enzymol. 178: 476-515 (1989); Skerra, A. et al., Biotechnology 9: 273-278 (1991)). The mutant proteins can be expressed for secretion in the medium and/or in the cytoplasm of the bacteria, as described by M. Better and A. Horwitz, Meth. Enzymol. 178: 476 (1989). In one disclosure, the single domains encoding VH and VL are each attached to the 3' end of a sequence encoding a signal sequence, such as the ompA, phoA or pelB signal sequence (Lei, S. P. et al., J. Bacteriol. 169: 4379 (1987)). These gene fusions are assembled in a dicistronic construct, so that they can be expressed from a single vector, and secreted into the periplasmic space of E. coli where they will refold and can be recovered in active form. (Skerra, A. et al., Biotechnology 9: 273-278 (1991)). For example, antibody heavy chain genes can be concurrently expressed with antibody light chain genes to produce antibody or antibody fragments.
In still another embodiment of the invention, the polynucleotides can be expressed in eukaryotic cells such as yeast using, for example, yeast display as described, e.g., in U.S. Patent Nos. 6,423,538; 6,331,391; and 6,300,065. In this approach, the polypeptide analogs of the library are fused to a polypeptide that is expressed and displayed on the surface of the yeast. Other eukaryotic cells for expression of the polypeptides of the disclosure can also be used such as mammalian cells, for example myeloma cells, hybridoma cells, or Chinese hamster ovary (CHO) cells. Typically, the polypeptide analogs when expressed in mammalian cells are designed to be expressed into the culture medium, or expressed on the surface of such a cell. The antibody or antibody fragments can be produced, for example, as entire antibody molecules or as individual VH and VL fragments, Fab fragments, single domains, or as single chains (sFv) (see Huston, J. S. et al, Proc. Natl. Acad. Sci. USA 85: 5879-5883 (1988)).

The screening of the expressed polypeptide analogs (or polypeptides produced by direct synthesis) can be done by any appropriate means. For example, binding activity can be evaluated by standard immunoassay and/or affinity chromatography and catalytic activity can be ascertained by suitable assays for substrate conversion. Screening of the polypeptide analogs of the disclosure for proteolytic function can be accomplished using a standard hemoglobin plaque assay as described, for example, in U.S. Patent No. 5,798,208.

### 5. Computer Model ing-Assisted Look Through Mutagenesis

The look-through mutagenesis of the disclosure may also be conducted with the benefit of structural or modeling information concerning the polypeptide analogs to be generated, such that the potential for generating analogs having the desired improved function is increased. The structural or modeling information can also be used to guide the selection of predetermined amino acid to introduce into the defined regions. Still further, actual results obtained with the polypeptide analogs of the disclosure can guide the selection (or exclusion) of subsequent polypeptides to be made and screened in an iterative manner. Accordingly, structural or modeling information can be used to generate initial subsets of polypeptide analogs for use in the disclosure, thereby further increasing the efficiency of generating improved polypeptides.

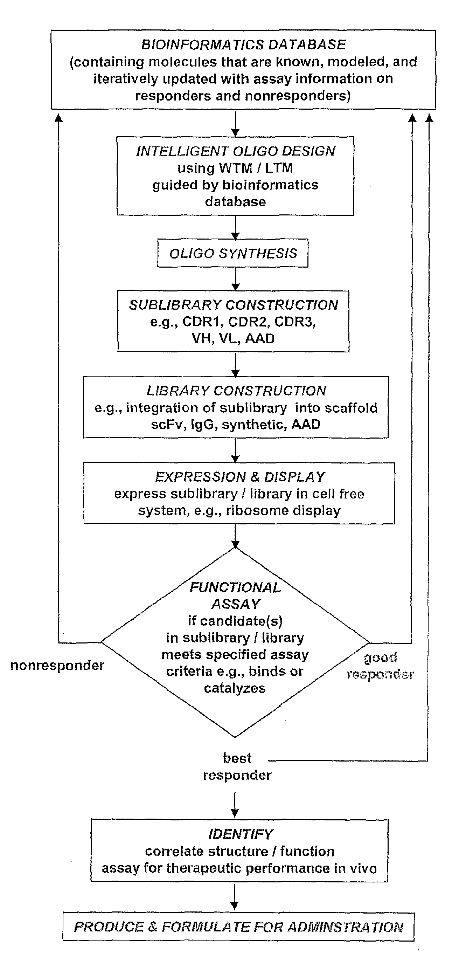

In a particular disclosure, in silico modeling is used to eliminate the production of any polypeptide analog predicted to have poor or undesired structure and/or function. In this way, the number of polypeptide analogs to be produced can be sharply reduced thereby increasing signal-to-noise in subsequent screening assays. In another particular disclosure, the in silico modeling is continually updated with additional modeling information, from any relevant source, e.g., from gene and protein sequence and three-dimensional databases and/or results from previously tested analogs, so that the in silico database becomes more precise in its predictive ability (Fig. 19).
In yet another disclosure the in silico database is provided with the assay results of previously tested polypeptide analogs and categorizes the analogs, based on the assay criterion or criteria, as responders or nonresponders, e.g., as polypeptide analogs that bind well or not so well or as being enzymatic/catalytic or not so enzymatic/catalytic, in this way, the guide-through mutagenesis of the disclosure can equate a range of functional response with particular structural information and use such information to guide the production of future polypeptide analogs to be tested. Accordingly, the method is especially suitable for screening antibody or antibody fragments for a particular function, such as binding affinity (e.g., specificity), stability (e.g., half life) and/or effector function (e.g., complement activation and ADCC). Accordingly, mutagenesis of non-contiguous residues within a region can be desirable if it is known, e.g., through in silico modeling, that certain residues in the region will not participate in the desired function. The coordinate structure and spatial interrelationship between the defined regions, e.g., the functional amino acid residues in the defined regions of the polypeptide, e.g., the predetermined amino acid(s) that have been introduced, can be considered and modeling. Such modeling criteria include, e.g., amino acid residue side group chemistry, atom distances, crystallography data, etc. Accordingly, the number of polypeptide analogs to be produced can be intelligently minimized.

In a preferred disclosure one or more of the above steps are computer- assisted. The method is also amenable to be carried out, in part or in whole, by a device, e.g., a computer driven device. Accordingly, instructions for carrying out the method, in part or in whole, can be conferred to a medium suitable for use in an electronic device for carrying out the instructions. In sum, the methods of the disclosure are amendable to a high throughput approach comprising software (e.g., computer-readable instructions) and hardware (e.g., computers, robotics, and chips).

### 6. Exploring the Combinatorial Chemistry of Multiple Defined Regions

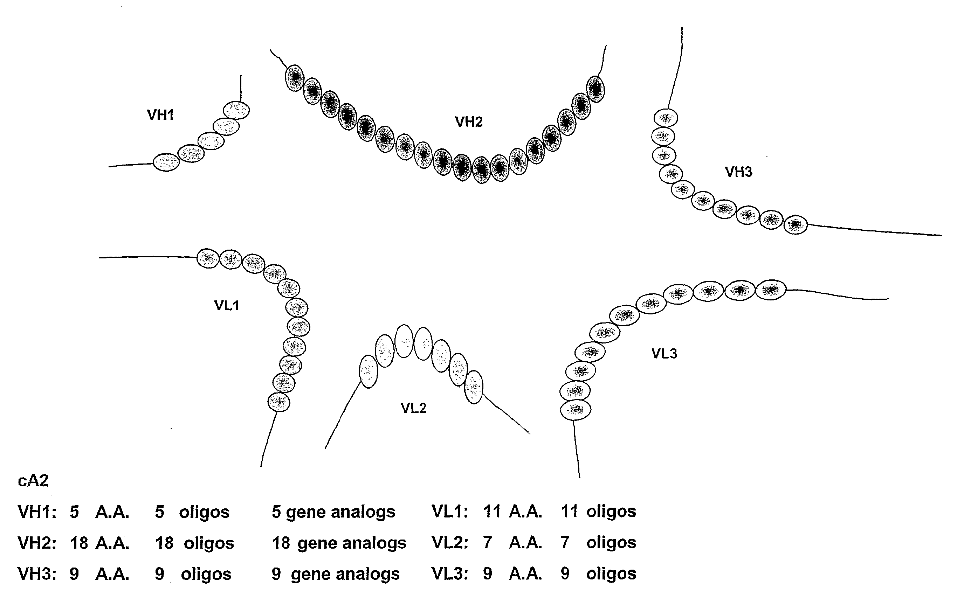

The present disclosure provides the important advantage of allowing for evaluation by mutagenesis of several different regions or domains of a polypeptide simultaneously. This can be done using the same or a different predetermined amino acid within each region, enabling the evaluation of amino acid substitutions in conformationally related regions, such as the regions that upon folding of the polypeptide, are associated to make up a functional site (e.g., the binding site of an antibody or the catalytic site of an enzyme). This, in turn, provides an efficient way to create new or improved functional sites.
For example, as depicted in Fig. 14, the six CDRs of an antibody that make up the unique aspects of the antigen binding site (Fv region), can be mutagenized simultaneously, or separately within the VH or VL chains, to study the three dimensional interrelationship of selected amino acids in this site. In one disclosure, the combinatorial chemistry of three or more defined regions are systematically explored using look-though mutagenesis, and preferably six defined regions, for example, the six CDRs of an antibody heavy and light chain variable region. For performing look- through mutagenesis on a CDR, typically 3, 4, 5, 6, 7, 8, 9, 10, 11,12, 13, 14, 15, 16, 17, 18, 19, 20, 21, 22, 23, 24, 25, or more amino acid positions are altered.

Accordingly, the present disclosure opens up new possibilities for the design of many different types of novel and improved polypeptides. The method can be used to improve upon an existing structure or function of a protein For example, a binding site for an antibody or antibody fragment can be introduced or affinity for a pre-existing antigen, effector function and/or stability improved. Alternatively, the introduction of additional "catalytically important" amino acids into a catalytic domain of an enzyme can be performed resulting in a modified or enhanced catalytic activity toward a substrate. Alternatively, entirely new structures, specificities or activities may be introduced into a polypeptide. De novo synthesis of enzymatic activity can be achieved as well. The new structures can be built on the natural or consensus "scaffold" of an existing protein by mutating only relevant regions by the method of the disclosure.

### 7. Look-Through Mutagenesis for Making New or Improved Antibodies

The method of this disclosure is especially useful for modifying antibody molecules. As used herein, antibody molecules or antibodies refers to antibodies or portions thereof, such as full-length antibodies, Fv molecules, or other antibody fragments, individual chains or fragments thereof (e.g., a single chain of Fv), single chain antibodies, and chimeric antibodies. Alterations can be introduced into the variable region and/or into the framework (constant) region of an antibody. Modification of the variable region can produce antibodies with better antigen binding properties, and, if desired, catalytic properties. Modification of the framework region can also lead to the improvement of chemo-physical properties, such as solubility or stability (e.g., half life), which are especially useful, for example, in commercial production, bioavailabilty, effector function (e.g., complement activation and/or ADCC) and binding affinity (e.g., specificity) for the antigen. Typically, the mutagenesis will target the Fv region of the antibody molecule, i.e., the structure responsible for antigen- binding activity which is made up of variable regions of two chains, one from the heavy chain (VH) and one from the light chain (VL). Once the desired antigen-binding characteristics are identified, the variable region(s) can be engineered into an appropriate antibody class such as IgG, IgM, IgA, IgD, or IgE.

### 8. Look-Through Mutagenesis for Making /Improving Catalytic /Enzymatic Polypeptides

The method of the disclosure also is particularly suited to the design of catalytic proteins, particularly catalytic antibodies. Presently, catalytic antibodies can be prepared by an adaptation of standard somatic cell fusion techniques. In this process, an animal is immunized with an antigen that resembles the transition state of the desired substrate to induce production of an antibody that binds the transition state and catalyzes the reaction. Antibody-producing cells are harvested from the animal and fused with an immortalizing cell to produce hybrid cells. These cells are then screened for secretion of an antibody that catalyzes the reaction. This process is dependent upon the availability of analogues of the transition state of a substrate. The process may be limited because such analogues are likely to be difficult to identify or synthesize in most cases.

The method of the disclosure provides a different approach that eliminates the need for a transition state analogue. By the method of the disclosure, an antibody can be made catalytic by the introduction of suitable amino acids into the binding site of an immunoglobulin (Fv region). The antigen-binding site (Fv) region is made-up of six hypervariable (CDR) loops, three derived from the immunoglobulin heavy chain (H) and three from the light chain (L), which connect beta strands within each subunit. The amino acid residues of the CDR loops contribute almost entirely to the binding characteristics of each specific monoclonal antibody. For instance, catalytic triads (comprising of amino acid residues serine, histidine, and aspartic acid) modeled after serine proteases can be created in the hypervariable segments of the Fv region of an antibody with known affinity for the substrate molecule and screened for proteolytic activity of the substrate.
In particular, the method of the disclosure can be used to produce many different enzymes or catalytic antibodies, including oxidoreductases, transferases, hydrolases, lyases, isomerases and ligases. Among these classes, of particular importance will be the production of improved proteases, carbohydrases, lipases, dioxygenases and peroxidases. These and other enzymes that can be prepared by the method of the disclosure have important commercial applications for enzymatic conversions in health care, cosmetics, foods, brewing, detergents, environment (e.g., wastewater treatment), agriculture, tanning, textiles, and other chemical processes. These include, but are not limited to, diagnostic and therapeutic applications, conversions of fats, carbohydrates and protein, degradation of organic pollutants and synthesis of chemicals. For example, therapeutically effective proteases with fibrinolytic activity, or activity against viral structures necessary for infectivity, such as viral coat proteins, can be engineered. Such proteases could be useful anti-thrombotic agents or anti- viral agents against viruses such as, for example, HIN, rhinoviruses, influenza, or hepatitis. In the case of oxygenases (e.g., dioxygenases), a class of enzymes requiring a co-factor for oxidation of aromatic rings and other double bonds, industrial applications in biopulping processes, conversion of biomass into fuels or other chemicals, conversion of waste water contaminants, bioprocessing of coal, and detoxification of hazardous organic compounds are possible applications of novel proteins.

The present invention is further illustrated in the following examples, which should not be construed as limiting.

### Exemplification

Throughout the examples, the following materials and methods were used unless otherwise stated.

### Materials and Methods

In general, the practice of the present invention employs, unless otherwise indicated, conventional techniques of chemistry, molecular biology, recombinant DNA technology, PCR technology, immunology (especially, e.g., antibody technology), expression systems (e.g., cell-free expression, phage display, ribosome display, and Profusion™), and any necessary cell culture that are within the skill of the art and are explained in the literature. See, e.g., Sambrook, Fritsch and Maniatis, Molecular Cloning: Cold Spring Harbor Laboratory Press (1989); DNA Cloning, Vols. 1 and 2, (D.N. Glover, Ed. 1985); Oligonucleotide Synthesis (M.J. Gait, Ed. 1984); PCR Handbook Current Protocols in Nucleic Acid Chemistry, Beaucage, Ed. John Wiley and Sons (1999) (Editor); Oxford Handbook of Nucleic Acid Structure, Neidle, Ed., Oxford Univ Press (1999); PCR Protocols: A Guide to Methods and Applications, Innis et al., Academic Press (1990); PCR Essential Techniques: Essential Techniques, Burke, Ed., John Wiley and Son Ltd (1996); The PCR Technique: RT-PCR, Siebert, Ed., Eaton Pub. Co. (1998); Antibody Engineering Protocols (Methods in Molecular Biology), 510, Paul, S., Humana Pr (1996); Antibody Engineering: A Practical Approach (Practical Approach Series, 169), McCafferty, Ed., hrl Pr (1996); Antibodies: A Laboratory Manual, Harlow et al., C.S.H.L. Press, Pub. (1999); Current Protocols in Molecular Biology, eds. Ausubel et al., John Wiley and Sons (1992); Large-Scale Mammalian Cell Culture Technology, Lubiniecki, A., Ed., Marcel Dekker, Pub., (1990). Phage Display: A Laboratory Manual, C. Barbas (Ed.), CSHL Press, (2001); Antibody Phage Display, P O'Brien (Ed.), Humana Press (2001); Border et al., Yeast surface display for screening combinatorial polypeptide libraries, Nature Biotechnology, 15(6):553-7 (1997); Border et al., Yeast surface display for directed evolution of protein expression, affinity, and stability, Methods Enzymol., 328:430-44 (2000); ribosome display as described by Pluckthun et al. in U.S. Patent No. 6,348,315, and Profusion™ as described by Szostak et al. in U.S. Patent Nos. 6,258,558; 6,261,804; and 6,214,553.

### EXAMPLE 1

### LOOK-THROUGH MUTAGENESIS OF THREE DEFINED REGIONS IN AN ANTIGEN BINDING

### MOLECULE

In this example, the look-through mutagenesis of three CDRs of an antibody to improve binding and proteolysis of a substrate is described.

In particular, the "look-through" mutagenesis of three complementarity determining regions (CDRs) of a monoclonal antibody is performed. CDR1, CDR2, and CDR3 of the heavy chain variable region (VH) are defined regions selected for look- through mutagenesis. For this disclosure, the predetermined amino acids selected are the three residues of the catalytic triad of serine proteases, Asp, His and Ser. Asp is selected for VH CDR1, His is selected for VL CDR2, and Ser is selected for VH CDR3. The selection of these three predetermined amino acids allows for the use of a convenient protease assay in order to detect when the three residues are positioned correctly to exhibit a functional activity, i.e., proteolysis of a test substrate.

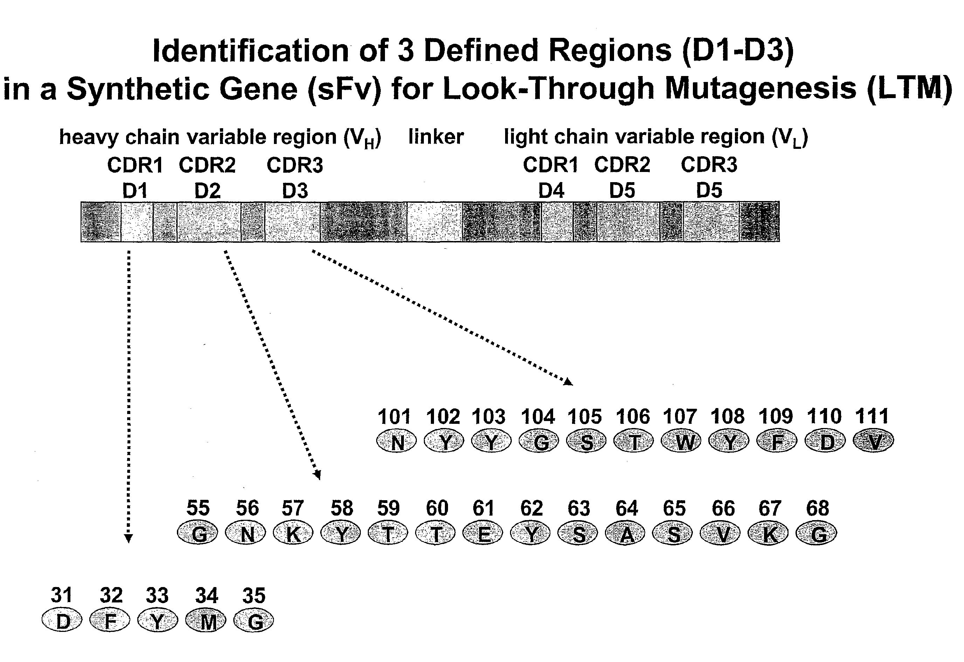

An exemplary antibody, MCPC 603, is recognized as a good model for investigating binding and catalysis because the antibody binding region has been well characterized. The amino acid sequence and DNA sequence of the MCPC 603 VH and VL regions are publicly available (see, e.g., Rudikoff, S. and Potter, M., Biochemistry 13: 4033 (1974); Pluckthun, A. et al., Cold Spring Harbor Symp. Quant. Biol., Vol. LII: 105-112 (1987)). The CDRs for the MCPC 603 antibody have been identified as shown in Fig. 2. In the heavy chain, CDR1 spans amino acid residue positions 31-35, CDR2 spans positions 50-69, and CDR3 spans positions 101-111. In the light chain, the amino acid residues of CDR1 are 24-40, CDR2 spans amino acids 55-62, and CDR3 spans amino acids 95-103.

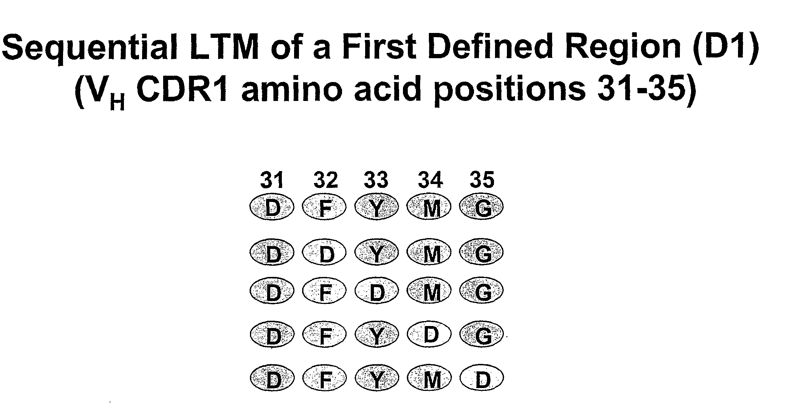

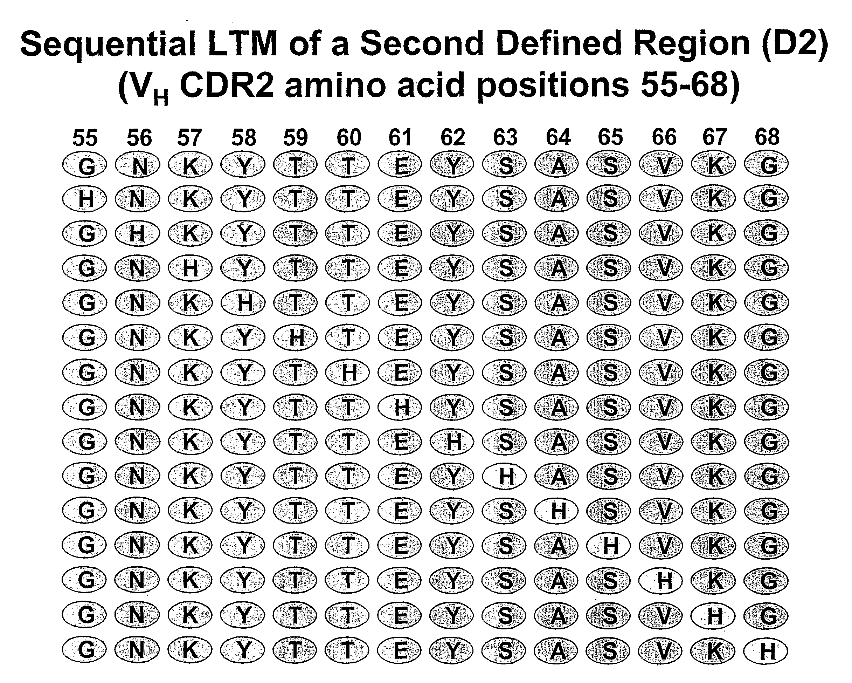

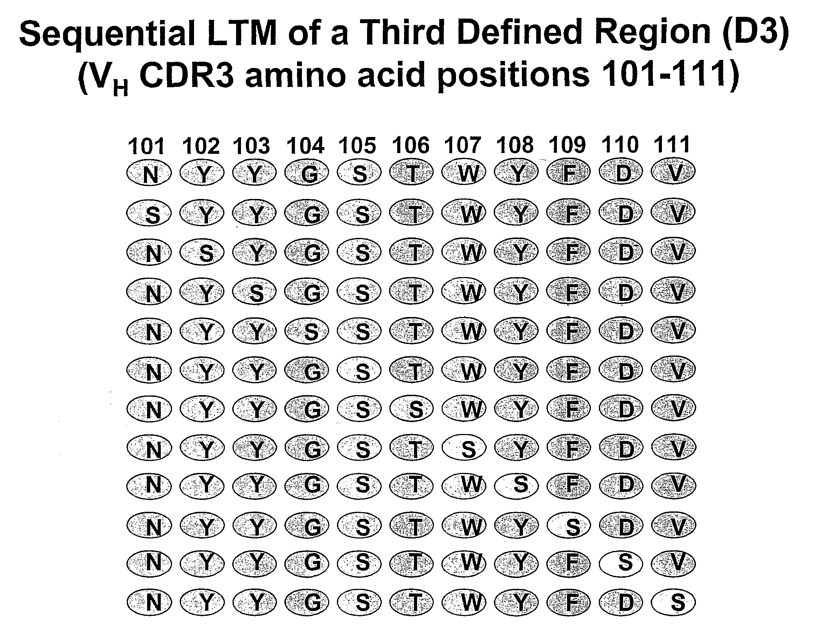

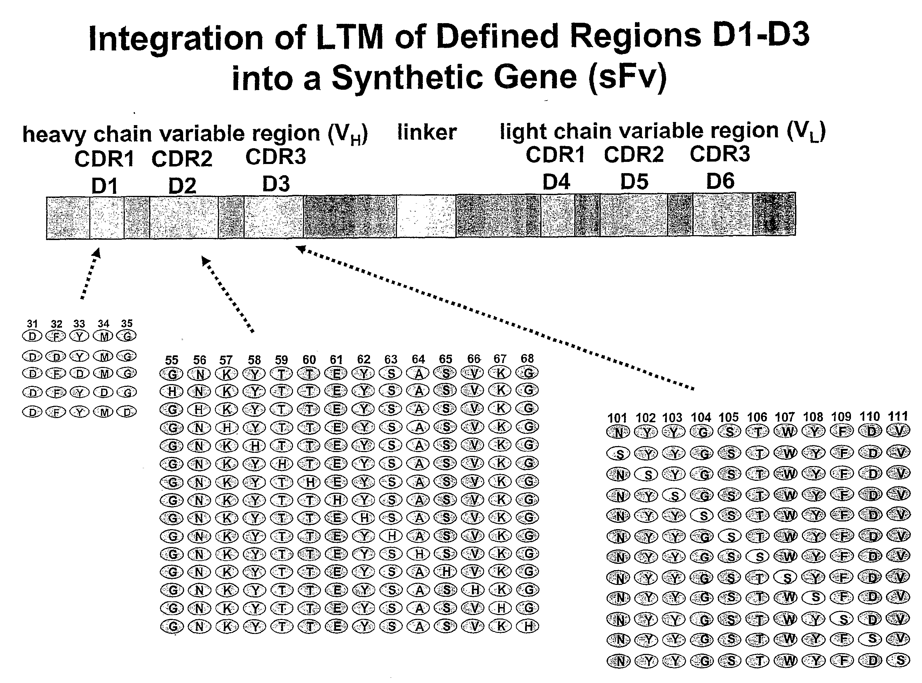

The design of the oligonucleotides for look-through mutagenesis in the CDRs of MCPC 603 is such that polypeptide analogs result having the amino acid sequence for CDR1, CDR2, and CDR3 as shown in Figs. 3-5. It is understood that the oligonucleotides synthesized can be larger than the CDR regions to be altered to facilitate insertion into the target construct as shown in Fig. 7. A single chain antibody format is chosen for convenience in subsequent expression and screening steps. The oligonucleotides can be converted into double-stranded chains by enzymatic techniques (see e.g., Oliphant, A. R. et al., 1986, supra) and then ligated into a restricted plasmid as shown in Fig. 8. The restriction sites can be naturally occurring sites or engineered restriction sites.

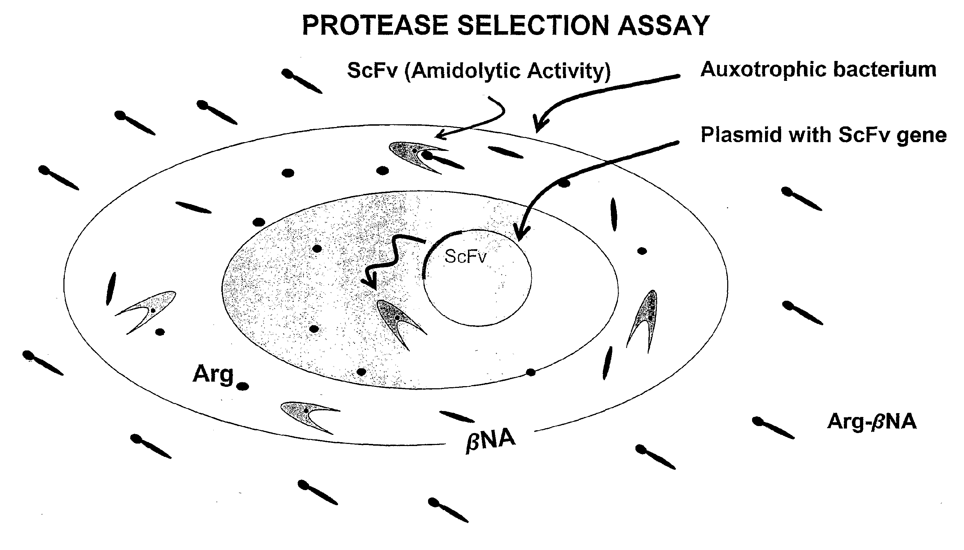

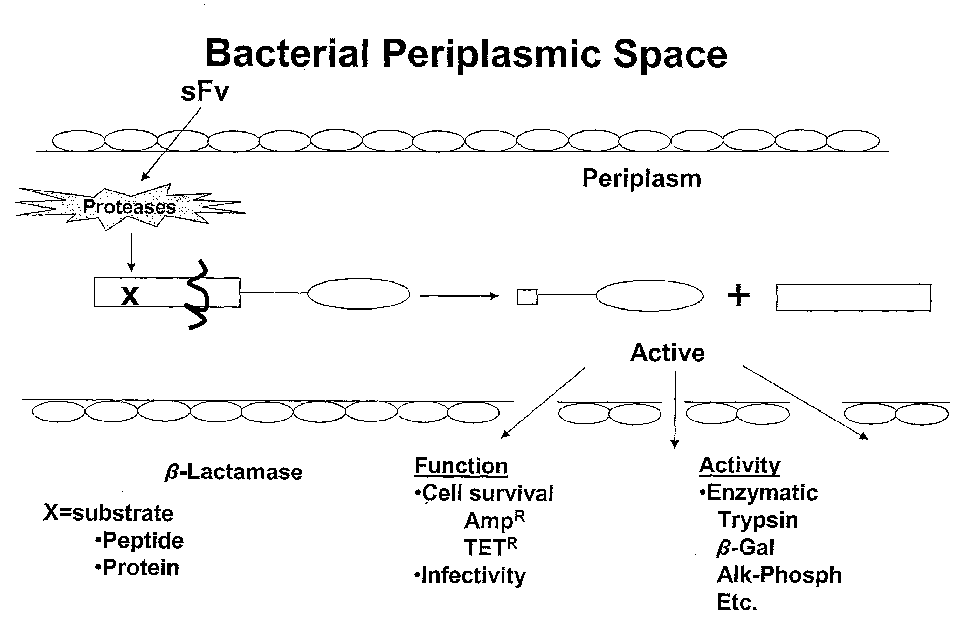

Polynucleotides encoding corresponding polypeptide analogs can be expressed in any of the convenient expression systems described herein and screened using the serine protease assay described, e.g., in U.S. Patent No. 5,798,208 (see also, Figs. 16-17). Briefly, the expressed polypeptide analogs are exposed to a test substrate and examined for proteolysis of the test substrate. The amount of proteolysis, revealed by a zone of clearance of the substrate, indicates a polypeptide analog having the desired functional activity.

### EXAMPLE 2

### LOOK-THROUGH MUTAGENESIS OF SIX DEFINED REGIONS IN AN ANTIGEN BINDING

### MOLECULE

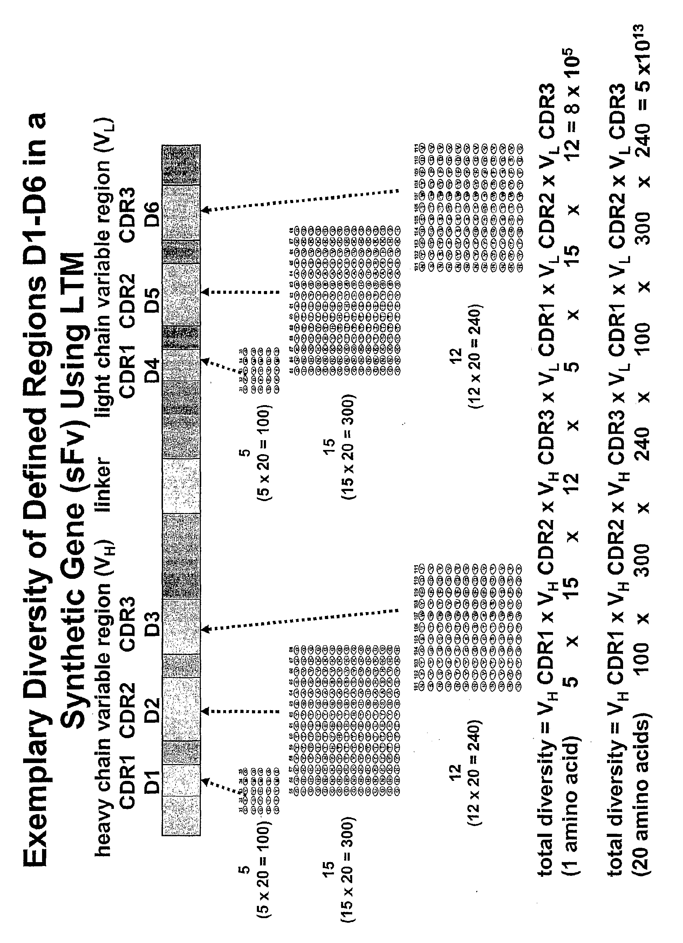

In this example, the look-through mutagenesis of all six CDRs of an antibody to improve binding and proteolysis of a substrate is described. In particular, a "look-through" mutagenesis of all six of the hypervariable regions or complementarity determining regions (CDRs) of the above mentioned model antibody (MCPC 603) is performed, in this example, "look-through" mutagenesis is carried out from two to three times with a different amino acid in a given region or domain. For example, Asp, Ser and His are sequentially walked-through the heavy and light chains as shown in Fig. 10.

Mutagenesis of noncontiguous residues within a region can be desirable if it is known, or if one can deduce, that certain residues in the region will not participate in the desired function. In addition, the number of analogs can be minimized. Other considerations in selecting the predetermined amino acid and the particular positions to be altered are that the residues must be able to hydrogen bond with one another. This consideration can impose a proximity constraint on the variants generated. Thus, only certain positions within the CDRs may permit the amino acids of the catalytic triad to interact properly. Thus, molecular modeling or other structural information can be used to enrich for functional variants.

In this case, known structural information was used to identify residues in the regions that may be close enough to permit hydrogen bonding between Asp, His and Ser, as well as the range of residues to be mutagenized. Roberts et al. have identified regions of close contact between portions of the CDRs (Roberts, V. A. et al., Proc. Natl. Acad. Sci. USA 87: 6654-6658 (1990)). This information together with data from the x- ray structure of MCPC 603 is used to select promising areas of close contact among the CDRs targeted for mutagenesis. This type of look-through mutagenesis guided by structural / modeling information can be referred to as "guide-through" mutagenesis.

Look-through mutagenesis is carried out as illustrated in Fig. 10 where each CDR is subjected to one predetermined amino acid resulting 8x10E5 polypeptide analogs and if all twenty amino acids are explored, 5x10E13 polypeptide analogs. Polynucleotides can be expressed in any of the convenient expression systems described herein and screened using the serine protease described, e.g., in U.S. Patent No.

5,798,208. Briefly, the expressed polypeptide analogs are exposed to a test substrate and examined for proteolysis of the test substrate. The amount of proteolysis, revealed by a zone of clearance of the substrate, indicates a polypeptide analog having the desired functional activity.

### EXAMPLE 3

### LOOK-THROUGH MUTAGENESIS OF ANTI-TNF BINDING MOLECULES TO IMPROVE

### FUNCTION

In this example, the look-through mutagenesis of an anti-TNF antibody to improve binding is described.

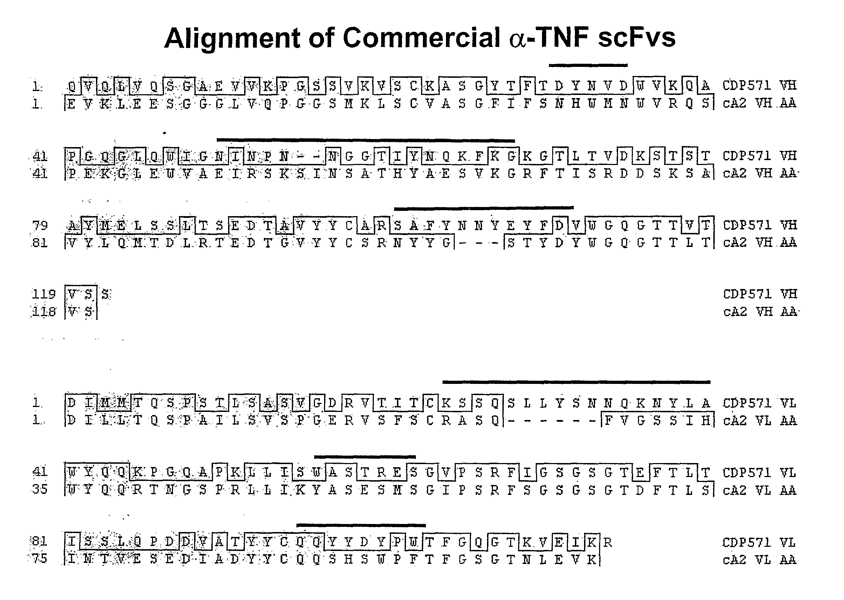

In particular, the "look-through" mutagenesis of all six of the hypervariable regions or complementarity determining regions (CDRs) of two different anti-TNF antibodies is performed. Anti-TNF antibodies have general application in the treatment of immune disease in patients having inappropriate levels of the ligand TNF (tumor necrosis factor). Two commercially available anti-TNF antibodies exist. For convenience in performing look-mutagenesis and subsequent screening, the variable light and heavy chain regions (see SEQ ID NOs: 2-4) of these antibodies were converted to a single chain format using a poly Gly-Ser linker (see Fig. 15). The defined regions selected for look through mutagenesis with a predetermined amino acid are identified by the presence of a black bar as shown in Fig. 15. These defined regions correspond to the CDRs of the single chain antibodies.

Polynucleotides representing the six CDR regions and sufficient flanking regions to allow for the assembly of the polynucleotides into the complete single chain sequence shown in Fig. 15 are synthesized as described herein. Predetermined amino acid residues are selected for each CDR region and separately and sequentially introduced into each amino acid position throughout the six CDR regions. The polynucleotides are further engineered to serve as templates capable of supporting the transcription of a corresponding RNA transcript that can then be translated into a polypeptide using ribosome display.

Polynucleotides encoding corresponding polypeptide analogs are expressed using a cell-free transcription and translation extracts. The RNA transcripts are covalently linked to a detectable moiety such as a fluorescent moiety. Alternatively, the sequence of interest can be fused in frame to a fluorescent moiety such as green fluorescent protein (GFP) to allow for the convenient detection of expression and normalization of binders versus non-binders. Preferably, the polynucleotides encoding the polypeptide analogs are at least partially arrayed, e.g., expressed in a well that has multiple polypeptide analogs but can be readily deconvoluted. Accordingly, each well contains a subset of polypeptide analogs now linked to the corresponding transcript linked to a florescent moiety using ribosome display. The well is probed with target ligand, i.e., TNF and assayed for polypeptide analogs that bind and with what binding affinity as compared to wild-type polypeptide. Polypeptide analogs that bind better than the wild-type polypeptide are then re- engineered into a full-length IgG antibody format for parallel testing with the analogous commercial antibody for improved binding using standard techniques.

### EXAMPLE 4

### LOOK-THROUGH MUTAGENESIS OF ANTIBODIES AGAINST BOTULINUM NERVE TOXIN

### SEROTYPE B (BoNT/B) AND BOTULINUM NERVE TOXIN SEROTYPE A (BoNT/A) TO

### IMPROVE FUNCTION

In this example, improved antibodies against botulinum nerve toxin serotype B (BoNT/B) and botulinum nerve toxin serotype A (BoNT/A) are generated using look- through mutagenesis (LTM) to improve function. The LTM approach is based on creating single mutations per CDR throughout the binding pocket, based on a subset (the LTM set) of the 20 amino acids that explore size, charge, hydrophobicity, and hydrogen bonding characteristics. The criteria for selecting the amino acids in the LTM set are discussed below.

### 1. Antibody Purification and Gene Sequencing and Single Chain (scFv) Design

Murine antibody fragments (Fab) with binding affinities to BoNT/B can be obtained as described in Emaneul et al. (1996) Journal of Immunological Methods 193: 189-197. The BotFab 5 (SEQ ID NOs: 10 and 11, light and heavy chain polypeptides, respectively), the BotFab 20 (SEQ ID NOs:12 and 13, light and heavy chain polypeptides, respectively) or the BotFab 22 (SEQ ID NOs:14 and 15, light and heavy chain polypeptides, respectively) antibodies can be used. The foregoing sequences are described in U.S. Patent No. 5,932,449,. The murine BoNT/B antigen (full-length toxin, light chain, and/or heavy chain) can be obtained from Metabiologics, Inc., WI.

The anti-BoNT/A antibodies described in Pless et al. (2001) Infect. Immun. 69:570 can be used with the objective of improving their affinities by at least one order of magnitude. The BoNT/A heavy chain binding domain (BoNT/A He) antigen can be obtained from Metabiologics, Inc., WI.

The V_{L} and V_{H} fragments of the antibody(ies) are cloned and sequenced using standard molecular biology techniques. The variable regions of the molecules are amplified by the polymerase chain reaction (PCR) and linked with a poly-Gly-Ser linker (typically SGGGGSGGGGSGGGGS (SEQ ID NO:7)) to generate single chain antibodies (scFv). A poly-His tag (HHHHHH (SEQ ID NO:8)) and a myc tag (EQKLISEEDL (SEQ ID NO:9)) are also appended to the C-terminus of the genes to facilitate purification and detection. These molecules are displayed on any of the well known technologies (e.g., yeast, bacterial or phage based technologies) and tested for their ability to bind BoNT/B or BoNT/A.

The monovalent scFv version of whole antibodies provide for a good format to undertake mutagenesis studies and are known to generally reproduce the binding mechanism of the whole molecule, with the exception of the avidity effects displayed by multivalent molecules.

### 2. Antibody Improvement Using Look Through Mutagenesis (LTM) and Gene Design

For the Look Through Mutagenesis (LTM), the following nine amino acids and their representative functional characteristics are chosen: Alanine and Leucine (aliphatics), Serine (hydroxyl group), Aspartic Acid (acidic) and Glutamine (amide), Lysine and Histidine (basic), Tyrosine (aromatic), Proline (hydrophobic). These amino acids display adequate chemical diversity in size, charge, hydrophobicity, and hydrogen bonding ability to provide meaningful initial information on the chemical functionality needed to improve antibody properties. The choices are also based on the frequency of occurrence of these amino acids in the CDRs of antibodies. For example, given between tyrosine and phenylalanine to represent amino acids with aromatic side chains, the former is chosen because of its significantly higher preponderance in antibody CDRs and its ability to hydrogen bond. LTM is initially employed to identify specific amino acids and chemical properties that are beneficial for binding, neutralization and/or any additional properties desired in the final antibody. However, LTM is not limited to these nine amino acids or nine total amino acids. LTM analysis can be performed with any combination of amino acids and the LTM subset can be as high as 18 amino acids (1 short of saturation mutagenesis).

### 2.1 LTM Oligonucleotide Synthesis

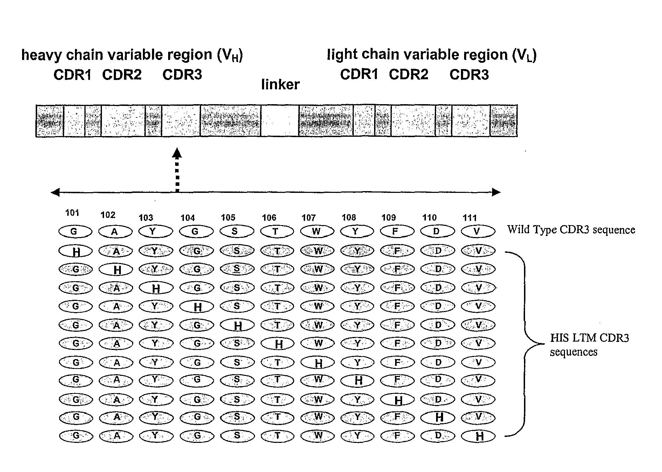

In the primary LTM analysis, the goal is to explore each amino acid's side chain contribution to the overall binding affinity within each CDR. To efficiently generate meaningful diversity, each of the LTM subset amino acid is targeted at every single position within the CDR sequence with only one substitution per CDR. Thus, each individual oligonucleotide encodes for only a single CDR mutation. For instance, in order to do LTM Histidine mutagenesis on a hypothetical eleven amino acid V_{H} CDR3 domain, 11 oligonucleotides are synthesized that encode for all 11 possible single Histidine mutations (see Figure 20). Such an analysis tests the effects of having a bulky amide in each position in the CDR. Therefore to generate a V_{H} CDR3 LTM library, only 99 oligonucleotides are synthesized for the LTM analysis (9 LTM amino acids x 11 V_{H} CDR3 positions). Oligonucleotide sequences are tested for inadvertent stop codons, wild-type duplication, inefficient codon usage, hairpins, loops, and other secondary structures using publicly available software.

### 2.2 Degenerate oligonucleotide synthesis for combinations of beneficial LTM mutations

In employing systematic LTM replacement of individual amino acids within a CDR, the preference of chemical functionalities at each position is uncovered. In order to combine all the mutations from the LTM selections and explore possible additivity and energetic synergy between these, degenerate oligonucleotides encoding for these mutations and the wild-type sequence are synthesized. This degenerate pool of oligonucleotides with 1.6x10⁴ variants is subsequently used to generate a second generation library that explores the additive nature of these substitutions.

### 2.3 Computer Assisted Oligonucleotide Design, Library, and Results Database

Software coupled with automated custom-built DNA synthesizers enables rapid oligonucleotide synthesis. The first step involves deciding which target amino acids will be incorporated into the CDRs. The software determines the codon preference (e.g., yeast, bacterial or phage codon preference, depending on the chosen system) needed to introduce the targeted amino acids and also eliminates any duplication of the wild-type sequence that may be generated by this design process. It then analyzes for potential stop codons, hairpins, and loop structures or for other problematic sequences that are subsequently corrected prior to synthesis. The completed LTM design plan is then sent to the DNA synthesizer, which performs an automated synthesis of the oligonucleotides. In this manner, the oligonucleotides needed to create the libraries can be rapidly generated.

An electronic database may store information of all LTM oligonucleotide sequences synthesized, details of the scFv library CDR substitutions, and binding assay results for a target antigen. Archival of the oligonucleotide data allows for rational iterations of the design strategies, and re-use of reagent oligonucleotides.

### 3. Overall LTM Strategy

LTM is initially employed to identify specific amino acids and chemical properties that are beneficial for binding, neutralization and/or any additional properties desired in the final antibody. It also quickly identifies the regions that do not tolerate any mutagenesis without significant loss in affinity (or any physical property being selected). Therefore, not only is the LTM analysis a good methodology to explore the chemical requirements at each position in all CDRs of an antibody, but it also rapidly determines the amino acids absolutely required for antigen binding. After the identification of beneficial mutations by LTM, combinatorial mutagenesis schemes may be employed that firstly incorporate all these disparate amino acid mutations to generate multiply mutated CDRs. Additionally or alternatively, Walk Through" mutagenesis (WTM) may be used to probe the effects of multiple mutations of the same amino acid in a CDR (as described, for example, in U.S. Patent Nos: 5,830,650; 5,798,208).

### 4. LTM scFv Libraries

The LTM techniques described above are used to create pools of oligonucleotides with mutations in a single CDR of the light or heavy chain. These oligonucleotides are synthesized to include some of the surrounding framework to facilitate the overlap and hybridization during PCR. These pools of oligonucleotides are utilized to generate all possible V_{L} and V_{H} chains in which there are mutations in single, double, and triple CDRs (single, double, and triple combinations of CDR1, 2, and 3) using single overlap extension PCR (SOE-PCR) (as described by Horton et al. (1989) Gene 77:61-68). SOE-PCR is a fast and simple method for combining DNA fragments that does not require restriction sites, restriction endonucleases, or DNA ligase. In SOE- PCR two regions in the gene are first amplified by PCR using primers designed so that the PCR products share a complementary sequence at one end. Under PCR conditions, the complementary sequences hybridize, forming an overlap. The complementary sequences then act as primers, allowing extension by DNA polymerase to produce a recombinant molecule.

For example, to create the pool of V_{H} chains in which both CDR-H1 and CDR- H2 are mutated and CDR-H3 is wild-type, which is denoted as "110" (1 denotes a mutant CDR and 0 denotes a wild-type CDR), the CDR-H1 mutant genes are used as templates and SOE-PCR is conducted to link the CDR-H2 oligonucleotides to generate the doubly mutated pool (Figure 21). Considering that each CDR may be either wild- type or mutant, there are seven possible combinations (depicted by arrows in Figure 21) for each of the pools of V_{L} and V_{H} chains (not including the wild-type molecule "000"). Combining the seven VL and eight V_{H} pools creates 63 V_{L}-V_{H} non-wild-type combinations (scFvs), (Figure 21). Each of the 64 V_{L}-V_{H} combinations (including the wild-type sequence) is termed as a "subset" of the whole LTM scFv library ensemble. An scFv library ensemble is created for each amino acid selected for substitution. The number of amino acid sequences represented within each subset library depends on the length of the CDR, the amino acid sequence within the CDR, and the LTM oligonucleotide design strategy.

### 5. High-Throughput Library Screening and Improved Antibody Selection

A variety of methods are available for antibody expression and display. These include bacteriophage, Escherichia coli, and yeast. While each of these methods has been used for antibody improvement, the yeast display system affords several advantages (Boder and Wittrup (1997) Nat. Biotechnol. 15:553-557). Yeast can readily accommodate library sizes up to 10⁷, with 10³-10⁵ copies of each antibody being displayed on each cell surface. Yeast cells are easily screened and separated using flow cytometry and fluorescence-activated cell sorting (FACS) or magnetic beads. Yeast also affords rapid selection and re-growth. The eukaryotic secretion system and glycosylation pathways of yeast allow for a much larger subset of scFv molecules to be correctly folded and displayed on the cell surface than prokaryotic display systems. Yeast display coupled with directed evolution has been used to increase the K_{D} of an scFv antibody fragment for fluorescein to 48 fM, two orders of magnitude stronger binding than any previously reported monovalent ligand (Boder et al. (2000) PNAS 97: 10701-10705). The display system utilizes the a-agglutinin yeast adhesion receptor to display proteins on the cell surface. The proteins of interest, in the present case, anti-BoNT/B scFv LTM libraries or anti-BoNT/A scFv LTM libraries, are expressed as fusion partners with the Aga2 protein. These fusion proteins are secreted from the cell and become disulfide linked to the Agal protein, which is attached to the yeast cell wall (see Invitrogen, pYDI Yeast Display product literature). In addition, there are carboxyl terminal tags included which may be utilized to monitor expression levels and/or normalize binding affinity measurements.

Streptavidin coated magnetic beads (Spherotech) are used to screen and select for antibodies that bind to BoNT/B or BoNT/A with high affinity. This methodology employs high affinity antibody binding to biotinylated antigen which then binds to streptavidin coated beads in order to select for the yeast clones (Yeung and Wittrup (2002) Biotechnol. Prog 18:212-220) and Feldhaus et al. (2003) Nature Biotech. 21:163-170). The BoNT/B or BoNT/A polypeptide (Metabiologics) is biotinylated using standard protocols (Pierce) and screening is performed using methods well known in the art. Using equilibrium and kinetic based selection, antibodies with improved affinities are selected for from these libraries. The efficacy of each round of selection is monitored by analytical FACS (FACScan). In addition, relative binding affinities of individual molecules displayed on the yeast surface are measured by titrating the antigen. This allows for rapid identification of molecules with improved affinity. The scFv clones are then sequenced to identify beneficial mutations.

### 6. Generation of Soluble Antibodies for BIAcore Affinity Measurements

Antibodies of interest are sub-cloned into soluble expression systems (Pichia pastoris and/or E. coli) and soluble protein is generated. There are several commercially available vectors and cell lines for soluble antibody expression, including those from Invitrogen (e.g., pPIC9 for P. pastoris) and Novagen (pET20b for periplasmic expression in E. coli). These systems are routinely used to generate soluble single chain or full-length antibodies. The P. pastoris expression system (Invitrogen) routinely produces 1-5 mg per liter of soluble purified scFv. Purification of proteins is facilitated by the presence of a His-tag at the C-terminus of the molecule, in the case of single chains or by protein A or protein G columns for full-length antibodies. Soluble single chain and full-length antibodies are generated to obtain BIAcore affinity kinetic rate measurements. This step is necessary as high affinity scFv molecules on the yeast clone cell surface must be verified as a cell free soluble molecule. Soluble single chain and full-length antibodies generated in the foregoing manner may be used to obtain BIAcore affinity measurements, as well as in the neurite outgrowth assay or the mouse lethality assay described below.

### 7. Screening of Selected Antibodies for In Vivo or In Vitro Neutralization

A cell-based assay using the neurite outgrowth of primary chick neurons as an indicator of BoNT intoxication and, thus, as a means to quantify toxin neutralization may be used to screen the selected antibodies. Preliminary experiments using dorsal root ganglion explant cultures from fertilized chicken eggs have indicated that there was less axonal outgrowth from explants treated with BoNT/A than the controls. Alternatively, a chick ciliary ganglion-iris muscle neuromuscular junction assay (as described in Lomneth et al. (1990) Neuroscience Letters 113:211-216) may be used.

The mouse lethality assay (MLA, as described by, for example, Schantz and Kautter (1978) J. Assoc. Off. Anal. Chem. 61:96-99) is another well known and accepted in vivo method for testing for BoNT neutralization. The test involves the interperitoneal injection of approximately 0.5 ml of sample preparations of BoNT/B or BoNT/A with and without the antibody into 20 to 30 gm, white ICR strain mice. Lethality due to respiratory failure is noted over 1-4 days. Quantification of neutralization requires serial dilutions of the MAbs with varying levels of mouse BoNT/B or BoNT/A LD₅₀. The MLA may be used to determine the neutralizing ability of the optimized antibodies identified by the in vitro screening.

The neutralizing ability of antibodies can also be measured in vitro by the mouse protection assay (MPA, Goeschel et al. (1997) Exp. Neurol. 147:96-102). In the MPA the left phrenic nerve, together with the left hemidiaphragm, is excised from the mouse.

The phrenic nerve is then continuously electrostimulated in a tissue bath. Purified antibodies are incubated with BoNT/B or BoNT/A, and added to the tissue bath. Toxin induced paralysis is defined as a 50 percent reduction in the initial muscle twitch.

The invention described herein is encompassed by the following claims.

## Claims

1. A yeast display expression polypeptide library comprising polynucleotides encoding anti-botulinum nerve toxin serotype B (BoNT/B) antibody single chain variable fragment (scFv) analogs, wherein each scFv comprises
(a) a poly-Gly-Ser linker of SEQ ID NO:7,
(b) a poly-His tag of SEQ ID NO:8,
(c) a myc tag of SEQ ID NO:9
(d) a VH of SEQ ID NO:10 and VL of SEQ ID NO:11 wherein the complementarity determining region (CDR) residues are substituted at each amino acid position within the six CDRs, the polynucleotides collectively representing all possible variant combinations according to the following criteria:
i) each polynucleotide contains at each codon position in each of the CDRs, either a codon required for the wild type amino acid residue of the polypeptide or a codon for the predetermined amino acid residue, and
ii) each polynucleotide contains no more than one codon for the
predetermined amino acid residue in each CDR; wherein
the predetermined amino acid residue is selected from the group consisting of Alanine, Leucine, Serine, Aspartic acid, Glutamine, Lysine, Histidine, Tyrosine and Proline, and wherein the anti-BoNT/B scFv is expressed as a fusion partner with Aga2 protein.

## Patentansprüche

1. Hefe-Display-Expressions-Polypeptid-Bibliothek, die Polynucleotide umfasst, die Analoga des variablen Einzelkettenfragments (scFv) von Anti-Botulinum-Nerventoxin-Serotyp-B (BoNT/B)-Antikörper codieren, wobei jedes scFv umfasst:
(a) einen Poly-Gly-Ser-Linker mit SEQ ID NO:7,
(b) ein Poly-His-tag mit SEQ ID NO:8,
(c) ein myc-tag mit SEQ ID NO:9,
(d) eine VH mit SEQ ID NO:10 und eine VL mit SEQ ID NO:11, worin die Reste der komplementaritätsbestimmenden Region (CDR) an jeder Aminosäureposition innerhalb der sechs CDRs substituiert sind, wobei die Polynucleotide zusammen alle möglichen Variantenkombinationen gemäß den folgenden Kriterien darstellen:
i) jedes Polynucleotid enthält an jeder Codonposition in jeder der CDRs entweder ein Codon, das für den Wildtyp-Aminosäurerest des Polypeptids erforderlich ist, oder ein Codon für den vorbestimmten Aminosäurerest, und
ii) jedes Polynucleotid enthält nicht mehr als ein Codon für den vorbestimmten Aminosäurerest in jeder CDR; wobei der vorbestimmte Aminosäurerest aus der Gruppe, bestehend aus Alanin, Leucin, Serin, Asparaginsäure, Glutamin, Lysin, Histidin, Tyrosin und Prolin, ausgewählt ist und wobei das Anti-BoNT/B scFv als Fusionspartner mit Aga2-Protein exprimiert wird.

## Revendications

1. Banque d'expression de polypeptides présentés par des levures comprenant des polynucléotides codant pour des analogues du fragment variable à chaîne unique (scFv) d'un anticorps anti-neurotoxine botulique du sérotype B (BoNT/B), chaque scFv comprenant
(a) un lieur poly-Gly-Ser de SEQ ID NO : 7,
(b) un marqueur poly-His de SEQ ID NO : 8,
(c) un marqueur myc de SEQ ID NO : 9,
(d) un VH de SEQ ID NO : 10 et un VL de SEQ ID NO : 11, dans lesquels les résidus des régions déterminant la complémentarité (CDR) sont substitués au niveau de chaque position d'acide aminé au sein des six CDR, les polynucléotides représentant collectivement toutes les combinaisons variantes possibles selon les critères suivants :
i) chaque polynucléotide contient au niveau de chaque position de codon dans chacune des CDR, soit un codon requis pour le résidu d'acide aminé de type sauvage du polypeptide soit un codon pour le résidu d'acide aminé prédéterminé, et
ii) chaque polynucléotide ne contient pas plus d'un codon pour le résidu d'acide aminé prédéterminé dans chaque CDR ; dans laquelle
le résidu d'acide aminé prédéterminé est choisi dans le groupe constitué d'alanine, leucine, sérine, acide aspartique, glutamine, lysine, histidine, tyrosine et proline, et dans laquelle le scFv de l'anticorps anti-BoNT/B est exprimé sous la forme d'un partenaire de fusion avec la protéine Aga2.


### SEQUENCE LISTING

<110> R. Crea & Co., et *al.*
<120> LOOK-THROUGH MUTAGENESIS
<130> BRI-001PC
<150> 60/483,282
   <151> 2003-06-27
<160> 13
<170> FastSEQ for Windows Version 4.0
<210> 1
   <211> 121
   <212> PRT
   <213> Artificial Sequence
<220>
   <223> Synthetic construct
<400> 1
   Gln Val Gln Leu Val Gln Ser Gly Ala Glu Val Val Lys Pro Gly Ser
    1               5                  10                  15
   Ser Val Lys Val Ser Cys Lys Ala Ser Gly Tyr Thr Phe Thr Asp Tyr
               20                  25                  30
   Asn Val Asp Trp Val Lys Gln Ala Pro Gly Gln Gly Leu Gln Trp Ile
           35                  40                  45
   Gly Asn Ile Asn Pro Asn Asn Gly Gly Thr Ile Tyr Asn Gln Lys Phe
       50                  55                  60
   Lys Gly Lys Gly Thr Leu Thr Val Asp Lys Ser Thr Ser Thr Ala Tyr
   65                  70                  75                  80
   Met Glu Leu Ser Ser Leu Thr Ser Glu Asp Thr Ala Val Tyr Tyr Cys
                   85                  90                  95
   Ala Arg Ser Ala Phe Tyr Asn Asn Tyr Glu Tyr Phe Asp Val Trp Gly
               100                 105                 110
   Gln Gly Thr Thr Val Thr Val Ser Ser
           115                 120
<210> 2
   <211> 119
   <212> PRT
   <213> Artificial Sequence
<220>
   <223> Synthetic construct
<400> 2
   Glu Val Lys Leu Glu Glu Ser Gly Gly Gly Leu Val Gln Pro Gly Gly
    1               5                  10                  15
   Ser Met Lys Leu Ser Cys Val Ala Ser Gly Phe Ile Phe Ser Asn His
               20                  25                  30
   Trp Met Asn Trp Val Arg Gln Ser Pro Glu Lys Gly Leu Glu Trp Val
           35                  40                  45
   Ala Glu Ile Arg Ser Lys Ser Ile Asn Ser Ala Thr His Tyr Ala Glu
       50                  55                  60
   Ser Val Lys Gly Arg Phe Thr Ile Ser Arg Asp Asp Ser Lys Ser Ala
   65                  70                  75                  80
   Val Tyr Leu Gln Met Thr Asp Leu Arg Thr Glu Asp Thr Gly Val Tyr
                   85                  90                  95
   Tyr Cys Ser Arg Asn Tyr Tyr Gly Ser Thr Tyr Asp Tyr Trp Gly Gln
               100                 105                 110
   Gly Thr Thr Leu Thr Val Ser
           115
<210> 3
   <211> 114
   <212> PRT
   <213> Artificial Sequence
<220>
   <223> Synthetic construct
<400> 3
   Asp Ile Met Met Thr Gln Ser Pro Ser Thr Leu Ser Ala Ser Val Gly
    1               5                  10                  15
   Asp Arg Val Thr Ile Thr Cys Lys Ser Ser Gln Ser Leu Leu Tyr Ser
               20                  25                  30
   Asn Asn Gln Lys Asn Tyr Leu Ala Trp Tyr Gln Gln Lys Pro Gly Gln
           35                  40                  45
   Ala Pro Lys Leu Leu Ile Ser Trp Ala Ser Thr Arg Glu Ser Gly Val
       50                  55                  60
   Pro Ser Arg Phe Ile Gly Ser Gly Ser Gly Thr Glu Phe Thr Leu Thr
   65                  70                  75                  80
   Ile Ser Ser Leu Gln Pro Asp Asp Val Ala Thr Tyr Tyr Cys Gln Gln
                   85                  90                  95
   Tyr Tyr Asp Tyr Pro Trp Thr Phe Gly Gln Gly Thr Lys Val Glu Ile
               100                 105                 110
   Lys Arg
<210> 4
   <211> 107
   <212> PRT
   <213> Artificial Sequence
<220>
   <223> Synthetic construct
<400> 4
   Asp Ile Leu Leu Thr Gln Ser Pro Ala Ile Leu Ser Val Ser Pro Gly
    1               5                  10                  15
   Glu Arg Val Ser Phe Ser Cys Arg Ala Ser Gln Phe Val Gly Ser Ser
               20                  25                  30
   Ile His Trp Tyr Gln Gln Arg Thr Asn Gly Ser Pro Arg Leu Leu Ile
           35                  40                  45
   Lys Tyr Ala Ser Glu Ser Met Ser Gly Ile Pro Ser Arg Phe Ser Gly
       50                  55                  60
   Ser Gly Ser Gly Thr Asp Phe Thr Leu Ser Ile Asn Thr Val Glu Ser
   65                  70                  75                  80
   Glu Asp Ile Ala Asp Tyr Tyr Cys Gln Gln Ser His Ser Trp Pro Phe
                   85                  90                  95
   Thr Phe Gly Ser Gly Thr Asn Leu Glu Val Lys
               100                 105
<210> 5
   <211> 16
   <212> PRT
   <213> Artificial Sequence
<220>
   <223> Synthetic construct
<400> 5
   Ser Gly Gly Gly Gly Ser Gly Gly Gly Gly Ser Gly Gly Gly Gly Ser
    1               5                  10                  15
<210> 6
   <211> 6
   <212> PRT
   <213> Artificial Sequence
<220>
   <223> Synthetic construct
<400> 6
   His His His His His His
    1               5
<210> 7
   <211> 10
   <212> PRT
   <213> Artificial Sequence
<220>
   <223> Synthetic construct
<400> 7
   Glu Gln Lys Leu Ile Ser Glu Glu Asp Leu
   1 5 10
<210> 8
   <211> 236
   <212> PRT
   <213> Artificial Sequence
<220>
   <223> Synthetic construct
<400> 8
   Met Lys Tyr Leu Leu Pro Thr Ala Ala Ala Gly Leu Leu Leu Leu Ala
   1 5 10 15
   Ala Gln Pro Ala Met Ala Asp Ile Gln Met Thr Gln Ser Pro Ala Ser
   20 25 30
   Leu Ser Ala Ser Val Gly Glu Thr Val Thr Ile Thr Cys Arg Ala Ser
   35 40 45
   Gly Asn Ile His Asn Tyr Leu Ala Trp Tyr Gln Gln Lys Gln Gly Lys
   50 55 60
   Ser Pro Gln Leu Leu Val Tyr Asn Ala Lys Thr Leu Ala Asp Gly Val
   65 70 75 80
   Pro Ser Arg Phe Ser Gly Ser Gly Ser Gly Thr Gln Tyr Ser Leu Lys
   85 90 95
   Ile Asn Ser Leu Gln Pro Glu Asp Phe Gly Ser Tyr Tyr Cys Gln His
   100 105 110
   Phe Trp Ser Thr Pro Trp Thr Phe Gly Gly Gly Thr Lys Leu Glu Ile
   115 120 125
   Lys Arg Ala Asp Ala Ala Pro Thr Val Ser Ile Phe Pro Pro Ser Ser
   130 135 140
   Glu Gln Leu Thr Ser Gly Gly Ala Ser Val Val Cys Phe Leu Asn Asn
   145 150 155 160
   Phe Tyr Pro Lys Asp Ile Asn Val Lys Trp Lys Ile Asp Gly Ser Glu
   165 170 175
   Arg Gln Asn Gly Val Leu Asn Ser Trp Thr Asp Gln Asp Ser Lys Asp
   180 185 190
   Ser Thr Tyr Ser Met Ser Ser Thr Leu Thr Leu Thr Lys Asp Glu Tyr
   195 200 205
   Glu Arg His Asn Ser Tyr Thr Cys Glu Ala Thr His Lys Thr Ser Thr
   210 215 220
   Ser Pro Ile Val Lys Ser Phe Asn Arg Asn Glu Cys
   225 230 235
<210> 9
   <211> 254
   <212> PRT
   <213> Artificial Sequence
<220>
   <223> Synthetic construct
<400> 9
   Met Lys Tyr Leu Leu Pro Thr Ala Ala Ala Gly Leu Leu Leu Leu Ala
    1               5                  10                  15
   Ala Gln Pro Ala Met Ala Glu Val Gln Leu Gln Gln Ser Gly Ala Glu
               20                  25                  30
   Leu Val Lys Pro Gly Ala Ser Val Lys Leu Ser Cys Thr Ala Ser Gly
           35                  40                  45
   Phe Asn Ile Lys Asp Thr Phe Met His Trp Val Lys Gln Arg Pro Glu
       50                  55                  60
   Gln Gly Leu Glu Trp Ile Gly Arg Ile Asp Pro Ala Asn Gly Asn Thr
   65                  70                  75                  80
   Glu Tyr Asp Pro Lys Phe Gln Gly Lys Ala Thr Ile Thr Ala Asp Thr
                   85                  90                  95
   Ser Ser Asn Thr Val Asn Leu Gln Leu Ser Ser Leu Thr Ser Glu Asp
               100                 105                 110
   Thr Ala Val Tyr Tyr Cys Ala Ser Gly Gly Glu Leu Gly Phe Pro Tyr
           115                 120                 125
   Trp Gly Gln Gly Thr Leu Val Thr Val Ser Ala Ala Lys Thr Thr Pro
       130                 135                 140
   Pro Ser Val Tyr Pro Leu Ala Pro Gly Ser Ala Ala Gln Thr Asn Ser
   145                 150                 155                 160
   Met Val Thr Leu Gly Cys Leu Val Lys Gly Tyr Phe Pro Glu Pro Val
                   165                 170                 175
   Thr Val Thr Trp Asn Ser Gly Ser Leu Ser Ser Gly Val His Thr Phe
               180                 185                 190
   Pro Ala Val Leu Gln Phe Asp Leu Tyr Thr Leu Ser Ser Ser Val Thr
           195                 200                 205
   Val Pro Ser Ser Thr Trp Pro Ser Glu Thr Val Thr Cys Asn Val Ala
       210                 215                 220
   His Pro Ala Ser Ser Thr Lys Val Asp Lys Lys Ile Val Pro Arg Asp
   225                 230                 235                 240
   Cys Thr Ser Gly Gly Gly Gly Ser His His His His His His
                   245                 250
<210> 10
   <211> 236
   <212> PRT
   <213> Artificial Sequence
<220>
   <223> Synthetic construct
<400> 10
   Met Lys Tyr Leu Leu Pro Thr Ala Ala Ala Gly Leu Leu Leu Leu Ala
    1               5                  10                  15
   Ala Gln Pro Ala Met Ala Asp Ile Gln Met Thr Gln Ser Pro Ala Ser
               20                  25                  30
   Leu Ser Ala Ser Val Gly Glu Thr Val Thr Ile Thr Cys Arg Ala Ser
           35                  40                  45
   Gly Asn Ile His Asn Tyr Leu Ala Trp Tyr Gln Gln Lys Gln Gly Lys
       50                  55                  60
   Ser Pro Gln Leu Leu Val Tyr Asn Ala Lys Thr Leu Ala Asp Gly Val
   65                  70                  75                  80
   Pro Ser Arg Phe Ser Gly Ser Gly Ser Gly Thr Gln Tyr Ser Leu Lys
                   85                  90                  95
   Ile Asn Ser Leu Gln Pro Glu Asp Phe Gly Ser Tyr Tyr Cys Gln His
               100                 105                 110
   Phe Trp Ser Thr Pro Trp Thr Phe Gly Gly Gly Thr Lys Leu Glu Ile
           115                 120                 125
   Lys Arg Ala Asp Ala Ala Pro Thr Val Ser Ile Phe Pro Pro Ser Ser
       130                 135                 140
   Glu Gln Leu Thr Ser Gly Gly Ala Ser Val Val Cys Phe Leu Asn Asn
   145                 150                 155                 160
   Phe Tyr Pro Lys Asp Ile Asn Val Lys Trp Lys Ile Asp Gly Ser Glu
                   165                 170                 175
   Arg Gln Asn Gly Val Leu Asn Ser Trp Thr Asp Gln Asp Ser Lys Asp
               180                 185                 190
   Ser Thr Tyr Ser Met Ser Ser Thr Leu Thr Leu Thr Lys Asp Glu Tyr
           195                 200                 205
   Glu Arg His Asn Ser Tyr Thr Cys Glu Ala Thr His Lys Thr Ser Thr
       210                 215                 220
   Ser Pro Ile Val Lys Ser Phe Asn Arg Asn Glu Cys
   225                 230                 235
<210> 11
   <211> 254
   <212> PRT
   <213> Artificial Sequence
<220>
   <223> Synthetic construct
<400> 11
   Met Lys Tyr Leu Leu Pro Thr Ala Ala Val Gly Leu Leu Leu Leu Ala
    1               5                  10                  15
   Ala Gln Pro Ala Met Ala Glu Val Gln Leu Gln Gln Ser Gly Ala Glu
               20                  25                  30
   Leu Val Lys Pro Gly Ala Ser Val Lys Leu Ser Cys Thr Ala Ser Gly
           35                  40                  45
   Phe Asn Ile Lys Asp Thr Phe Met His Trp Val Lys Gln Arg Pro Glu
       50                  55                  60
   Gln Gly Leu Glu Trp Ile Gly Arg Ile Asp Pro Ala Asn Gly Asn Thr
   65 70 75 80
   Glu Tyr Asp Pro Lys Phe Gln Gly Lys Ala Thr Ile Thr Ala Asp Thr
   85 90 95
   Ser Ser Asn Thr Val Asn Leu Gln Leu Ser Ser Leu Thr Ser Glu Asp
   100 105 110
   Thr Ala Val Tyr Tyr Cys Ala Ser Gly Gly Glu Leu Gly Phe Pro Tyr
   115 120 125
   Trp Gly Gln Gly Thr Leu Val Thr Val Ser Ala Ala Lys Thr Thr Pro
   130 135 140
   Pro Ser Val Tyr Pro Leu Ala Pro Gly Ser Ala Ala Gln Thr Asn Ser
   145 150 155 160
   Met Val Thr Leu Gly Cys Leu Val Lys Gly Tyr Phe Pro Glu Pro Val
   165 170 175
   Thr Val Thr Trp Asn Ser Gly Ser Leu Ser Ser Gly Val His Thr Phe
   180 185 190
   Pro Ala Val Leu Gln Ser Asp Leu Tyr Thr Leu Ser Ser Ser Val Thr
   195 200 205
   Val Pro Ser Ser Thr Trp Pro Ser Glu Thr Val Thr Cys Asn Val Ala
   210 215 220
   His Pro Ala Ser Ser Thr Lys Val Asp Lys Lys Ile Val Pro Arg Asp
   225 230 235 240
   Cys Thr Ser Gly Gly Gly Gly Ser His His His His His His
   245 250
<210> 12
   <211> 236
   <212> PRT
   <213> Artificial Sequence
<220>
   <223> Synthetic construct
<400> 12
   Met Lys Tyr Leu Leu Pro Thr Ala Ala Ala Gly Leu Leu Leu Leu Ala
    1               5                  10                  15
   Ala Gln Pro Ala Met Ala Asp Ile Gln Met Thr Gln Ser Pro Ala Ser
               20                  25                  30
   Leu Ser Ala Ser Val Gly Glu Thr Val Thr Ile Thr Cys Arg Ala Ser
           35                  40                  45
   Gly Asn Ile His Asn Tyr Leu Ala Trp Tyr Gln Gln Lys Gln Gly Lys
       50                  55                  60
   Ser Pro Gln Leu Leu Val Tyr Asn Ala Lys Thr Leu Ala Asp Gly Val
   65                  70                  75                  80
   Pro Ser Arg Phe Ser Gly Ser Gly Ser Gly Thr Gln Tyr Ser Leu Lys
                   85                  90                  95
   Ile Asn Ser Leu Gln Pro Glu Asp Phe Gly Ser Tyr Tyr Cys Gln His
               100                 105                 110
   Phe Trp Ser Thr Pro Trp Thr Phe Gly Gly Gly Thr Lys Leu Glu Ile
           115                 120                 125
   Lys Arg Ala Asp Ala Ala Pro Thr Val Ser Ile Phe Pro Pro Ser Ser
       130                 135                 140
   Glu Gln Leu Thr Ser Gly Gly Ala Ser Val Val Cys Phe Leu Asn Asn
   145                 150                 155                 160
   Phe Tyr Pro Lys Asp Ile Asn Val Lys Trp Lys Ile Asp Gly Ser Glu
                   165                 170                 175
   Arg Gln Asn Gly Val Leu Asn Ser Trp Thr Asp Gln Asp Ser Lys Asp
               180                 185                 190
   Ser Thr Tyr Ser Met Ser Ser Thr Leu Thr Leu Thr Lys Asp Glu Tyr
           195                 200                 205
   Glu Arg His Asn Ser Tyr Thr Cys Glu Ala Thr His Lys Thr Ser Thr
       210                 215                 220
   Ser Pro Ile Val Lys Ser Phe Asn Arg Asn Glu Cys
   225                 230                 235
<210> 13
   <211> 254
   <212> PRT
   <213> Artificial Sequence
<400> 13
   Met Lys Tyr Leu Leu Pro Thr Ala Ala Ala Gly Leu Leu Leu Leu Ala
    1               5                  10                  15
   Ala Gln Pro Ala Met Ala Glu Val Gln Leu Gln Gln Ser Gly Ala Glu
               20                  25                  30
   Leu Val Lys Pro Gly Ala Ser Val Lys Leu Ser Cys Thr Ala Ser Gly
           35                  40                  45
   Phe Asn Ile Lys Asp Thr Phe Met His Trp Val Lys Gln Arg Pro Glu
       50                  55                  60
   Gln Gly Leu Glu Trp Ile Gly Arg Ile Asp Pro Ala Asn Gly Asn Thr
   65                  70                  75                  80
   Glu Tyr Asp Pro Lys Phe Gln Gly Lys Ala Thr Ile Thr Ala Asp Thr
                   85                  90                  95
   Ser Ser Asn Thr Val Asn Leu Gln Leu Ser Ser Leu Thr Ser Glu Asp
               100                 105                 110
   Thr Ala Val Tyr Tyr Cys Ala Ser Gly Gly Glu Leu Gly Phe Pro Tyr
           115                 120                 125
   Trp Gly Gln Gly Thr Leu Val Thr Val Ser Ala Ala Lys Thr Thr Pro
       130                 135                 140
   Pro Ser Val Tyr Pro Leu Ala Pro Gly Ser Ala Ala Gln Thr Asn Ser
   145                 150                 155                 160
   Met Val Thr Leu Gly Cys Leu Val Lys Gly Tyr Phe Pro Glu Pro Val
                   165                 170                 175
   Thr Val Thr Trp Asn Ser Gly Ser Leu Ser Ser Gly Val His Thr Phe
               180                 185                 190
   Pro Ala Val Leu Gln Ser Asp Leu Tyr Thr Leu Ser Ser Ser Val Thr
           195                 200                 205
   Val Pro Ser Ser Thr Trp Pro Ser Glu Thr Val Thr Cys Asn Val Ala
       210                 215                 220
   His Pro Ala Ser Ser Thr Lys Val Asp Lys Lys Ile Val Pro Arg Asp
   225                 230                 235                 240
   Cys Thr Ser Gly Gly Gly Gly Ser His His His His His His
                   245                 250